# EUROPÄISCHE PATENTSCHRIFT

(11) **EP 0 210 412 B1**
(45) Veröffentlichungstag und Bekanntmachung des Hinweises auf die Patenterteilung: **13.12.1995**
(21) Anmeldenummer: 86108324.4
(22) Anmeldetag: 19.06.1986
(51) Int. Cl.: C07K 5/00, C07K 7/00, A61K 39/00, A61K 38/02, A61K 47/48, C07K 17/02, C12N 15/02, G01N 33/532, B01J 20/32

(54) **Membrananker-Wirkstoffkonjugat, seine Herstellung sowie seine Verwendung**
Anchoring membrane ingredient conjugate, and its use
Conjugué d'un agent d'ancrage membranaire et d'un agent actif, son obtention et son emploi

(30) Priorität: 24.06.1985 DE 3522512; 27.12.1985 DE 3546150
(43) Veröffentlichungstag der Anmeldung: 04.02.1987
(73) Patentinhaber: HOECHST AKTIENGESELLSCHAFT, 65926 Frankfurt am Main (DE)
(72) Erfinder: Jung, Günther, Prof. Dr., D-7400 Tübingen (DE); Wiesmüller, Karl-Heinz, D-7400 Tübingen (DE); Metzger, Jörg, D-7400 Tübingen (DE); Bühring, Hans-Jörg, Dr., D-7400 Tübingen (DE); Becker, Gerhard, Dr., D-7404 Ofterdingen (DE); Bessler, Wolfgang, Prof. Dr., D-7401 Hagelloch (DE)

(56) Entgegenhaltungen:
- EP-A- 0 000 330
- EP-A- 0 014 815
- EP-A- 0 114 787
- LIEBIGS ANNALEN DER CHEMIE, Heft 9, 15. September 1983, Verlag Chemie GmbH, Weinheim (DE); G. JUNG et al., Seiten 1459-1467
- CHEMICAL ABSTRACTS, Band 101, Nr. 23, 03. Dezember 1984, Columbus, OH (US); W.G. BESSLER et al., Seite 701, Nr. 211690
- CHEMICAL ABSTRACTS, Band 99, Nr. 17, 24 Oktober 1983, Columbus, OH (US); R.B. JOHNSON et al., Seite 451, Nr. 138041q
- PEPTIDES, STRUCTURE & FUNCTION, Proceedings of the 9th Am. Peptide Symposium, Toronto, 23-28. Juni 1985, Pierce Chem. Co., Rockford, NJ (US); G. JUNG et al., Seiten 27-31
- CHEMICAL ABSTRACTS, Band 103, Nr. 21, 25. November 1985, Columbus, OH (US); G. JUNG et al., Seite 539, Nr. 176708w
- CHEMICAL ABSTRACTS, Band 104, Nr. 3, 20. Januar 1986, Columbus, OH (US); W.G. BESSLER et al., Nr. 18419u

## Beschreibung

Die Erfindung betrifft Membranankerwirkstoffkonjugate mit mindestens einem kovalent an die Membranankerverbindung (en) gebundenen Wirkstoff, Verfahren zu ihrer Herstellung sowie ihre Verwendung.

Membranankerverbindungen sind Verbindungen, die in biologische und künstliche Membranen einschleusbar sind.

Beispielsweise können derartige Membranankerverbindungen natürliche Membran-Lipoproteine sein, wie sie bereits aus der Außenmembran von Escherichia coli isoliert wurden und zwischenzeitlich auch synthetisiert worden sind. Die E. coli Membranankerverbindung besteht im N-terminalen Bereich aus drei Fettsäuren, welche an S-Glyceryl-L-cystein gebunden sind (G. Jung et al in "Peptides, Structure and Function", V. J. Hruby and D.H. Rich, Seiten 179 bis 182, Pierce Chem. Co. Rockford, Illinois, 1983).

Zur modellhaften Untersuchung von biologischen Membranen sind auch bereits konformations-stabilisierte Alpha-Helix-Polypeptide beschrieben worden, siehe unter anderem Alamethicin, ein Alpha-helicales amphiphiles Eicosapeptid-Antibiotikum, welches spannungsabhängige ionenleitende Systeme im Lipidmembranen bildet (Boheim, G., Hanke, W., Jung, G., Biophys. Struct. Mech. 9, Seiten 181 bis 191 (1983); Schmitt, H. und Jung, G., Liebigs Ann. Chem. Seiten 321 bis 344 und 345 bis 364 (1985).

In der EP-A1-330 ist beschrieben, daß Lipopeptide, die Analoga des bereits seit 1973 bekannten Lipoproteins aus E. coli sind, immunpotenzierend wirken. Eine weitere europäische Patentanmeldung, EP-A2-114787,befaßt sich mit der Fähigkeit von derartigen Lipopeptiden, Ratten- und Maus-Alveolar-Makrophagen in vitro zu aktivieren, so daß diese nach 24 stündiger Inkubation mit der Substanz Tumorzellen eliminieren können und insbesondere die Antikörperproduktion, beispielsweise gegen Schweineserumalbumin, signifikant steigern.

Es wird in EP-A2-114787 vorgeschlagen, diese Lipoproteinabkommlinge als Adjuvanzen bei der Immunisierung einzusetzen, also die Lipoproteinabkömmlinge des E. coli-Membranproteins in Mischung mit Antigenen zur Verbesserung der Immunantwort zu verwenden.

Es besteht ein großer Bedarf an die Immunantwort stimulierenden und verstärkenden Substanzen, insbesondere, da gereinigte Antigene häufig nur ingeringsten Mengen zugänglich sind; ferner ist bei Verwendung neuer Chargen von Antigenen stets die Möglichkeit von neuen Verunreinigungen bzw. Zersetzungsprodukten gegeben.

Ferner ist es erwünscht, ein Versuchstier nicht häufig impfen zu müssen, sondern durch möglichst einmalige Gabe des immunogenen Materials die erwünschte Immunantwort zu erhalten.

Es ist daher eine Aufgabe der vorliegenden Erfindung, die Antikörper-Bildung gegen Antigene bzw. Haptene zu steigern und somit eine spezifische immunpotenzierende Wirkung zu erhalten.

Die Aufgabe wird erfindungsgemäß durch das neue Membranankerwirkstoffkonjugat gelöst, mit mindestens einer Membranankerverbindung und mindestens einem kovalent an die Membranankerverbindung(en) gebundenen Wirkstoff.

Erfindungsgemäß wird auch ein Verfahren zum Herstellen von Membranankerverbindungen vorgeschlagen, welches dadurch gekennzeichnet ist, daß das mit Schutzgruppen in an sich bekannter Weise an den Funktionen, an denen keinerlei Reaktion stattfinden soll, geschützte Peptid mittels bekannter Kupplungsverfahren an einer festen oder löslichen Träger, wie einem Polymer (z.B. Merrifield-Harz) synthetisiert wird; daß die derart synthetisierten, am Träger gebundenen Peptide über N-Termini oder Seitenfunktionen des Peptids mit der Membranankerverbindung kovalent gebunden werden; daß das derart hergestellte Peptid-Konjugat isoliert wird dadurch daß die Schutzgruppen sowie die Bindung Peptid/Träger in an sich bekannter Weise gespalten werden und somit das Membranankerpeptid oder das Membranankerwirkstoffkonjugat gewonnen wird.

Die Erfindung bezieht sich auch auf die Verwendung der Verbindungen zur Herstellung konventioneller und monoklonaler Antikörper in vivo und in vitro; vorteilhafterweise lassen sich die erfindungsgemäßen Verbindungen aber auch in der Gentechnologie zur Erleichterung der Zellfusion, zur Herstellung synthetischer Vaccine, zur Herstellung von Zellmarkern mit Fluoreszenzlabeln, Spinlabeln, radioaktiven Labeln oder ähnlichem, fur die Affinitätschromatographie, insbesondere fur Affinitätssauler; für Liposomenpräparate; als Zusatz zu Lebensmitteln oder Futterstoffen im Human- und Tierbereich, sowie als Zusatz zu Kulturmedien für Mikroorganismen und allgemein für Zellkulturen einsetzen. Dabei können die erfindungsgemäßen Verbindungen gegebenenfalls gemeinsam mit an sich bekannten Trägern in Losung, Salben, adsorbiert an festen Trägern, in Emulsionen oder Sprays für human- oder tiermedizinische Zwecke angewendet werden.

Erfindungsgegenstand ist ein Membrananker-Wirkstoffkonjugat, dadurch gekennzeichnet, daß es aus mindestens einer Membranankerverbindung und mindestens einem kovalent an die Membranankerverbindung(en) gebundenen Wirkstoff besteht, wobei die Membranankerverbindung eine Verbindung der allgemeinen Formel I ist: 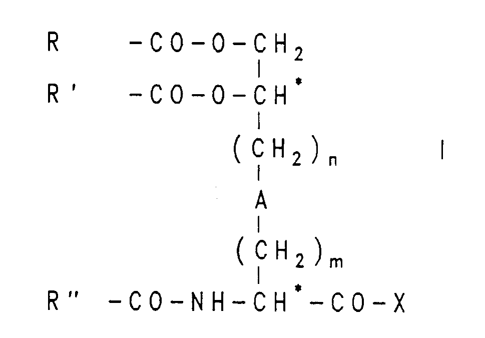
wobei A = Schwefel, Sauerstoff, Disulfid (-S-S-), Methylen (-CH₂-) oder -NH-sein kann; n = 0 bis 5; m = 1 oder 2; C* = asymmetrisches Kohlenstoffatom mit R oder S Konfiguration R, R', R'' gleich oder unterschiedlich sind und eine Alkyl-, Alkenyl- oder Alkinyl-Gruppe mit 7 bis 25 Kohlenstoffatomen oder Wasserstoff ist, welche gegebenenfalls mit Hydroxi-, Amino-, Oxo-, Acyl-, Alkyl- oder Cycloalkyl-Gruppen substituiert sein kann und X ein Wirkstoff oder eine Spacer-Wirkstoffgruppe ist
mit Ausnahme der Verbindungen der Formel I' 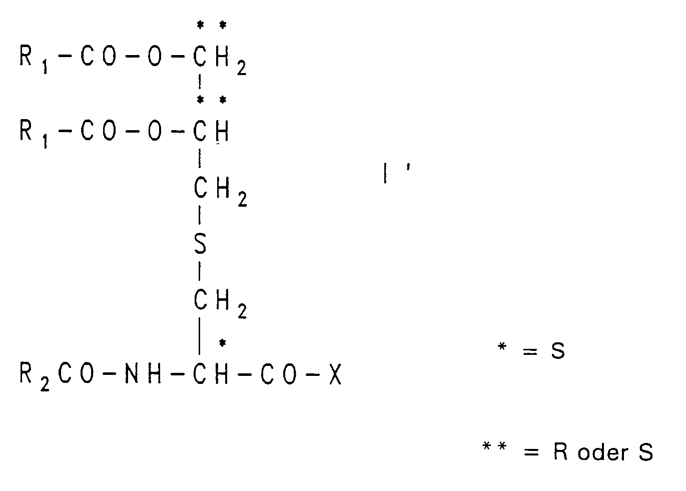
worin R₁ und R₂ je einen gesättigten oder ungesättigten, aliphatischen oder gemischt aliphatisch-cycloaliphatischen, gegebenenfalls auch durch Sauerstoffunktionen substituierten, Kohlenwasserstoffrest mit 11 bis 21 C-Atomen und Y eine peptidisch gebundene natürliche aliphatische Aminosäure mit freier, veresterter oder amidierter Carboxylgruppe, oder eine Aminosäurensequenz von 2 bis 10 natürlichen aliphatischen Aminosäuren, deren terminale Carboxylgruppe in freier, veresterter oder amidierter Form vorliegt, bedeuten, wobei die mit * bzw. ** bezeichneten Asymmetriezentren die absolute S- oder S- oder R-Konfiguration besitzen, und Gemische der an den ** C-Atomen epimeren R- und S-Verbindungen.

Bevorzugt ist ein Membrananker-Wirkstoffkonjugat wie oben beschrieben, dadurch gekennzeichnet, daß die Membranankerverbindung Pam₃Cys oder Pam₃Cys-Ser oder ein Pam₃Cys-Peptid mit 1 bis 10 Aminosäuren ist.

Es ist besonders bevorzugt, wenn die Membranankerwirkstoffkonjugate dadurch hergestellt werden, daß das mit Schutzgruppen in an sich bekannter Weise an den Funktionen, an denen keinerlei Reaktion stattfinden soll, geschützte Peptid mittels bekannter Kupplungsverfahren an einem festen oder löslichen Träger, wie ein Polymer (z.B. Merrifield-Harz) synthetisiert wird; daß die derart synthetisierten, am Träger gebundenen Peptide über N-Termini oder Seitenfunktionen des Peptids mit der Membranankerverbindung kovalent gebunden werden; daß das derart hergestellte Peptid-Konjugat isoliert wird, dadurch daß die Schutzgruppen sowie die Bindung Peptid/Träger in an sich bekannter Weise gespalten werden und somit das Membranankerpeptid oder das Membranankerwirkstoffkonjugat gewonnen wird.

Die Verknüpfung Peptid/Membranankerverbindung kann durch Kondensation, Addition, Substitution, Oxidation (bspw. Disulfidbildung) hergestellt werden.

Vorteilhaft kann sein, wenn der Wirkstoff mit zwei gegebenenfalls unterschiedlichen Membranankerverbindungen kovalent verknüpft ist.

Zusätzlich kann der Wirkstoff auch mit einem an sich bekannten Adjuvans für Immunisierungszwecke, wie z.B. Muramyldipeptid und/oder einem Lipopolysaccharid kovalent vernüpft sein.

Als Wirkstoffe schlagen wir beispielsweise vor : ein Antigen, wie beispielsweise eine niedermolekulare Partialsequenz eines Proteins oder Proteids, beispielsweise eines Glycoproteins, eines Virushüllproteins, Bakterienzellwandproteins oder Proteins von Protozoen (antigene Determinate, Epitop), Bakterienmembranbestandteile, wie Muramyldipeptid, Lipopolysaccharid, ein Antibiotikum, ein Hormon, ein Nukleosid, ein Nukleotid, eine Nukleinsäure, ein Enzym, ein Enzymsubstrat, ein Enzyminhibitor, Biotin, Avidin, Polyethylenglykol, Peptidwirkstoffe wie Tuftsin; Polylysin, ein Alkaloid, Steroid, biogenes Amin, Vitamin, ein Toxin wie beispielsweise Digoxin, Phalloidin, Amanitin oder Tetrodoxin.

Je nach Art des Wirkstoffes ergeben sich völlig neuartige Einsatzgebiete der erfindungsgemäßen Substanzen.

Es kann auch nützlich sein, wenn mehrere Membrananker-Wirkstoffkonjugatverbindungen im Lipid- und/oder Wirkstoffteil miteinander quervernetzt sind.

Die Membranankerverbindungen und der Wirkstoff können auch über einen Crosslinker miteinander verbunden sein, was zur Folge hat, daß der Wirkstoff sich mehr von der Membran entfernt, an die er durch den Membrananker befestigt wird.

Beispielsweise eignet sich als Crosslinker eine Dicarbonsäure oder ein Dicarbonsäurederivat, Diole, Diamine, Polyethylenglycol, Epoxide, Maleinsäurederivate oder dergl.

Erfindungsgemäß wird also ein eindeutig definiertes, niedermolekulares Konjugat, das sich u.a. zur Immunisierung eignet, hergestellt, das die Prinzipien Carrier-Antigen-Adjuvans kovalent miteinander verknüpft. Carrier und Adjuvans ein mitogen wirksames Lipopeptid, wie z.B. Tripalmitoyl-S-Glyceryl-Cystein (Pam3Cys) und dessen Analoga sein. Somit unterscheiden sich die neuen Verbindungen in wesentlichen Punkten von allen bisher eingesetzten höhermolekularen Konjugaten von Antigenen mit hochmolekularen Trägersubstanzen, beispielsweise Proteinen wie Serumalbuminen, Globulinen oder Polylysin oder allgemein höhermolekularen, linearen oder quervernetzten Polymeren.

Insbesondere unterscheiden sich die neuen Verbindungen aber auch von allen bisher bekannten Adjuvantien, die lediglich zugemischt werden und demzufolge keine gezielte Präsentation des Antigens an der Zelloberfläche ermöglichen. Die bisher bekannten Adjuvantien erforderten häufig mehrfache Immunisierungen und führten auch zu entzündlichen Reaktionen beim Tierversuch. Erfindungsgemäß ist ein besonderer Vorteil die reproduzierbare Herstellbarkeit pyrogenfreier, reiner, chemisch eindeutig definierter Verbindungen, die - im Gegensatz zu konventionellen Verbindungen bzw. Mischungen verschiedener Stoffe - auch zu besserer Reproduzierbarkeit der Antikörperbildung führt. Somit wird ein besonderer Einsatzbereich der erfindungsgemäßen Verbindungen im Bereich der Antikörpergewinnung, der Gentechnologie, der Herstellung synthetischer Vaccine, der Diagnostik und Therapie in Veterinär- und Humanmedizin gesehen, da die neuen Konjugate erstmals eine spezifisch die Immunantwort stimulierende Wirkung haben, während die bisher verwandten Adjuvantien lediglich unspezifisch die Immunantwort stimulierten. Überraschenderweise lassen sich durch die erfindungsgemäßen Verbindungen auch schwach immunogene Verbindungen in stark immunogene umwandeln. Somit liegt eine besondere Bedeutung der Erfindung in der Möglichkeit, Tierversuche sowie Kosten für die Herstellung von Antikörpern einzusparen, da die neuen Immunogene auch in vitro hochaktiv sind. Aufgrund der nicht entzündlichen Immunisierungsmethode kann ein Tier auch mehrfach für unterschiedliche Antikörpergewinnungen eingesetzt werden.

Schließlich könnten mit den neuen Immunogenen auch multivalente Impfstoffe hergestellt werden, d.h. beispielsweise ein Membrananker, an dessen Seitenketten mehrere Antigene oder Haptene angeknüpft sind, so daß mittels einer Immunisierung mehrere unterschiedliche aktive Antikörper hergestellt werden können.

Eine wasserlösliche, mitogene Lipidankergruppe ist beispielsweise Pam₃Cys-Ser(Lys)ₙ-OH, die sich insbesondere zur Herstellung der neuen Immunogene, aber auch zur Herstellung von fluoreszierenden, radioaktiven und biolo-gisch aktiven Zellmarkern eignet. Eine besondere erwünschte Eigenschaft der erfindungsgemäßen Membrananker-Wirkstoffkonjugate ist deren Amphiphilie, d.h.eine partielle Wasserlöslichkeit, da biologische Tests an Tieren und Untersuchungen mit lebenden Zellen dann wesentlich einfacher durchgeführt werden können. Auch die künstlichen Lipiddoppelschichtmembranen, Liposomen und Vesikel, die für manche Versuche benötigt werden, sind nur im wässrigen Medium herstellbar und stabil.

Ein geeigneter amphiphiler, biologisch aktiver Membrananker ist z.B. Pam₃Cys-Ser(Lys)ₙ-OH. Der an Pam₃Cys gekoppelte Serinrest begünstigt immunogene Eigenschaften, während die polaren, protonierten Epsilon-Aminogruppen der Lysin-Reste den hydrophilen Teil des Moleküls darstellen. Aufgrund seiner mehrfachen Ladungen weist dieser Verbindungstyp weitere interessante Eigenschaften auf. Er kann durch Induktion der Zellwechselwirkung als Fusionsaktivator bei der Herstellung von Hybridomzellen eingesetzt werden, insbesondere bei längerer Lysinkette, bei Kopplung an Polyethylenglycol oder bei kovalenter Inkorporation des Biotin/Avidinsystems.

Weiterhin können vorteilhafterweise die erfindungsgemäßen Verbindungen für die Herstellung neuartiger Liposomen durch Quervernetzung eingesetzt werden, die entweder in Fettsäure oder im Peptidteil stattfinden kann.

Die Membrananker (Pam₃Cys und Analoga, sowie die Helices) eignen sich ferner zur Verstärkung der Zell/Zellwechselwirkung, wenn sie beispielsweise mit dem Biotin/Avidinsystem kovalent kombiniert werden. Weitere vorteilhafte Eigenschaften der erfindungsgemäßen Verbindungen sind, daß sie Zellfusionen erleichtern können, wie sie beispielsweise für gentechnologische Arbeiten benötigt werden. Hierbei können die neuen Immunogene auch bei ELISA, RIA und Bioluminiszenzasssys eingesetzt werden.

Verschiedene Pam₃Cys-Derivate sind lipid- und wasserlöslich, und in vivo und in vitro stark mitogen wirksam. Sie eignen sich auch sehr gut zur Markierung von Zellen mit FITC und anderen Markern wie RITC, Dansyl und Cumarin. Insbesondere kann man sie auch bei der Fluoreszenzmikroskopie und dem Fluorescence Activated Cell Sorting (FACS) einsetzen.

Ein kostengünstiger Membrananker bei analoger Wirkung wie Pam₃Cys ist S-(1,2-Dioctadecyloxycarbonylethyl) -Cystein, dessen Herstellung im experimentellen Teil genauer beschrieben wird.

Spezifische Kopplungen der mitogen wirksamen Lipidanker an Antigene können auch über Crosslinker erfolgen, wie beispielsweise mit Dicarbonsäuremonohydrazidderivaten der allgemeinen Formel:

X-NH-NH-CO-A-CO-B-Y

oder auch

X-NH-NH-CO-A-COOH,

wobei A, B = Aminosäure oder (CH₂)ₙ und X, Y an sich bekannte Schutzgruppen sind.

Es kann auch jeder andere geeignete Crosslinker oder Spacer zur Herstellung der neuen Stoffe verwendet werden, wobei stets das Prinzip (niedermolekularer Carrier und Adjuvans)-(Antigen) eine besonders bevorzugte Ausgestaltung der Erfindung darstellt, sofern es Lipopeptidstrukturen mit Lipidmembranankerfunktion und/oder konformationsstabilisierte Helices beinhaltet.

Besonders vorteilhafte Wirkungen lassen sich durch Anwendung der erfindungsgemäßen Verbindungen in der Affinitätschromatographie finden, wofür sich insbesondere Lipopeptid-Antigen-(Hapten)-Konjugate eignen. Diese eignen sich in hervorragender Weise zum Beladen herkömmlicher Reversed-phase-HPLC-Säulen (oder auch präparativer RP-Säulen), wobei sich beispielsweise eine in organisch wässrigen Systemen aufgetragene Tripalmitoylverbindung in der apolaren Alkylschicht verankert. Das Antigen bleibt wie auf Zelloberflächen der mobilen wässrigen Phase präsentiert und lädt somit die Antikörper zur Adsorption ein. Somit können aus verdünntem Serum direkt auf einer derartigen Affinitätssäule Antikörper angereichert bzw. isoliert werden, die mit dem betreffenden Antigen spezifisch reagieren. Die Elution der Antikörper erfolgt wie bei anderen Affinitätssäulen, beispielsweise durch pH-Wert-Einstellung.

Im folgenden soll nun die Erfindung näher anhand von Beispielen erläutert werden, wobei zunächst die hier verwendeten Abkürzungen aufgeführt werden sollen:
Aib = 2-Methyl-alanin
TFE = Trifluoressigsäure
EGF R = Rezeptor des Epidermal Growth Factor
Pam = Palmitoyl-Rest
DCC = Dicyclohexyl-carbodiimid
DMF = Dimethylformamid
FITC = Fluoresceinisothiocyanat
Fmoc = Fluorenylmethoxycarbonyl
But = tert. Butylrest
PS - DVB = Styrol - Divinylbenzol-Copolymeres mit 4-(Hydroxymethyl)phenoxymethyl-Ankergruppen
HOBt = 1-Hydroxybenzotriazol
RITC = Rhodaminisothiocyanat
Hu IFN-(Ly) 11-20 = antigene Determinante von Human-Interferon
DCH = N,N'-Dicyclohexylharnstoff
EE = Ethylacetat
In den beiliegenden Figuren, die zur Erläuterung der Erfindung dienen, zeigt:

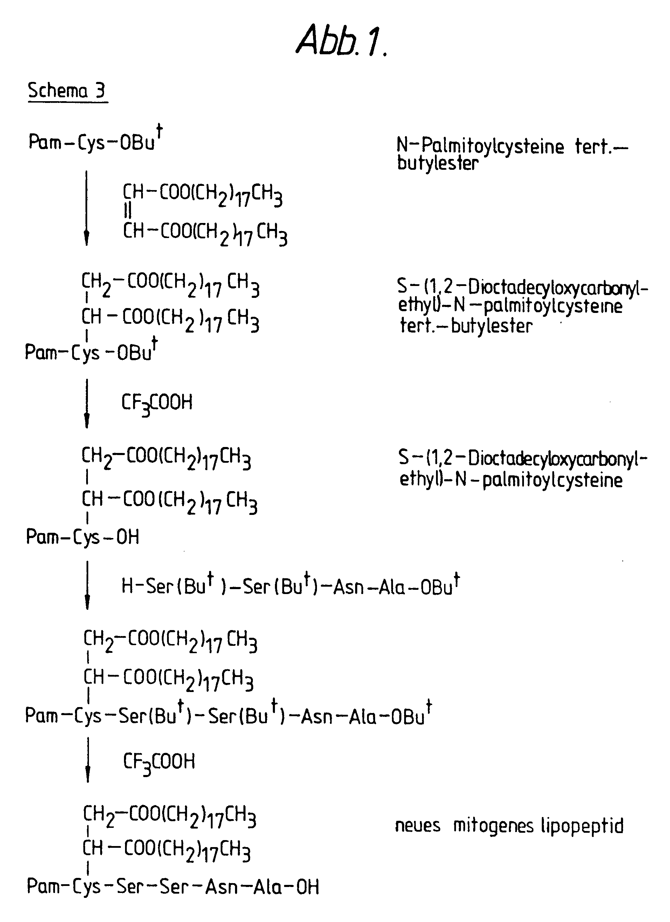

- Fig. 1: das Herstellungsschema von Pam-Cys(C₁₈)₂-Ser-Ser-Asn-Ala-OH
- Fig. 2: die Tabelle der ¹³C-NMR-Spektren

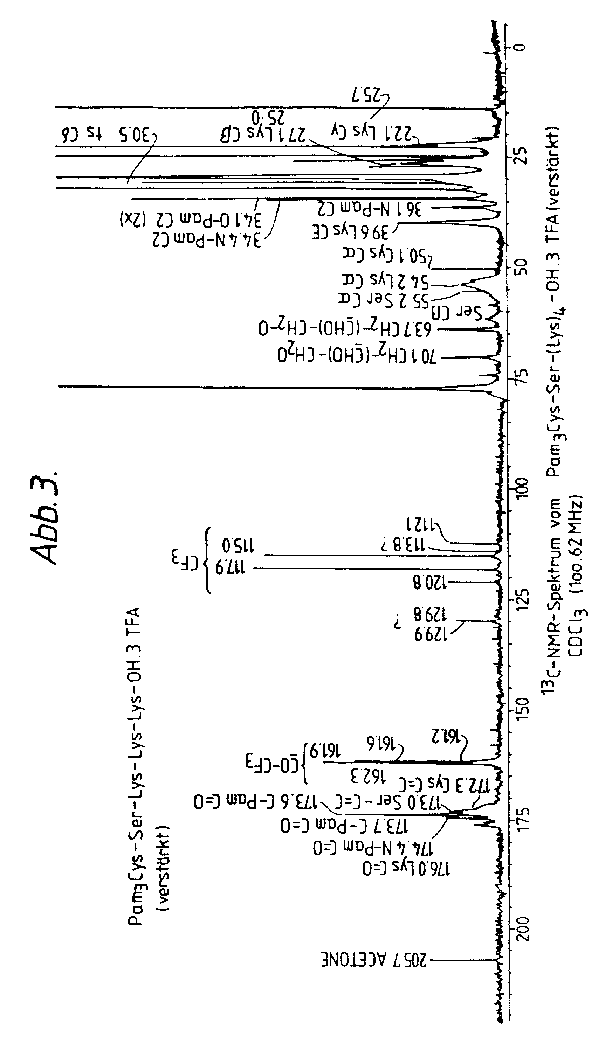

- Fig. 3: das ¹³C-NMR-Spektrum von Pam₃Cys-Ser-(Lys)₄-OH x 3 TFA in CDCl3

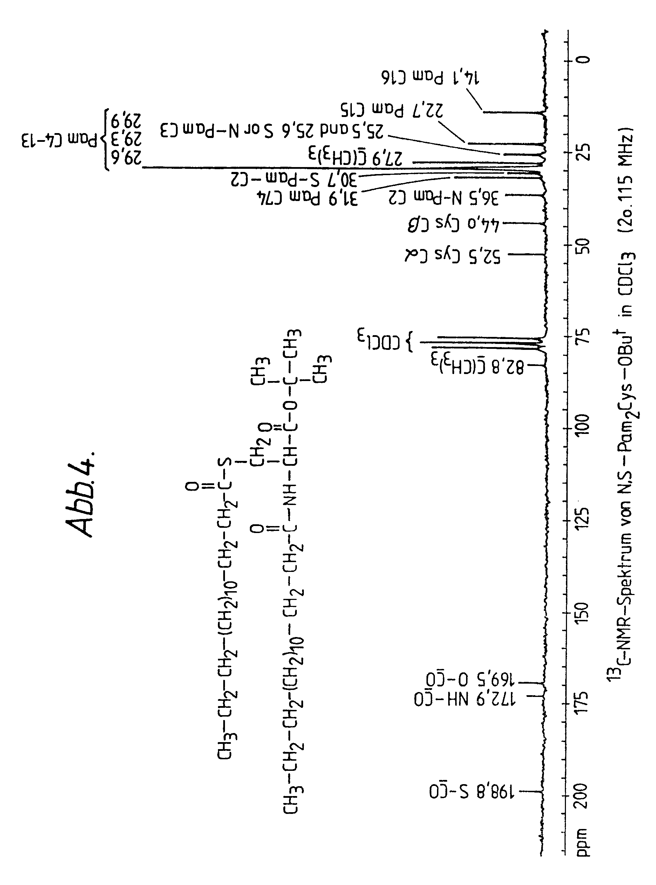

- Fig. 4: das ¹³C-NMR-Spektrum von Pam-Cys(Pam)-OBu^{t} in CDCl3

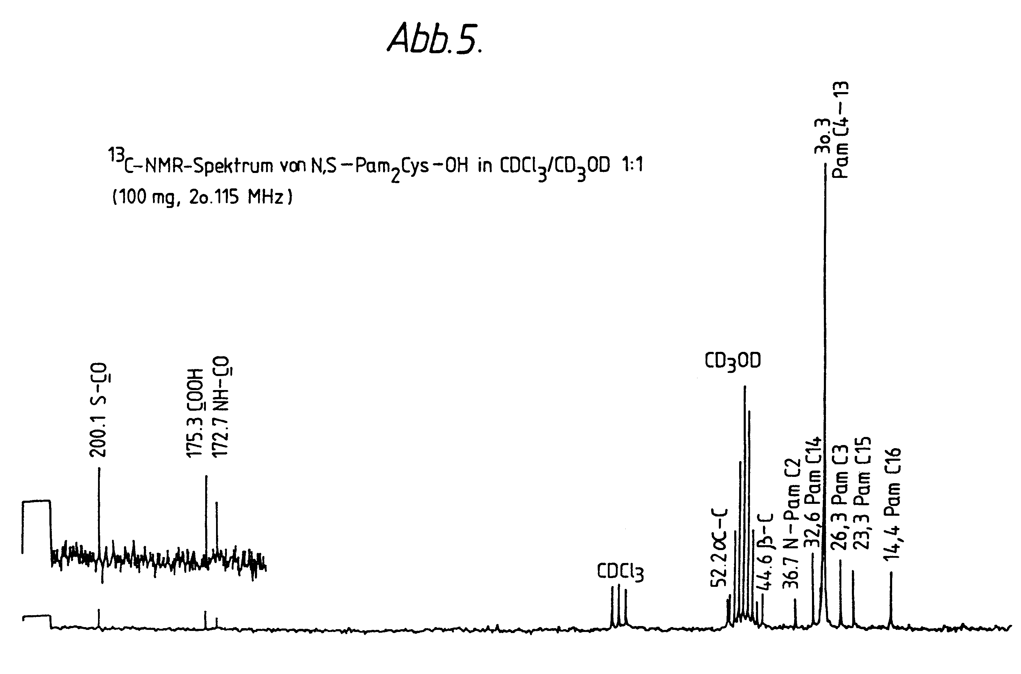

- Fig. 5: das ¹³C-NMR-Spektrum von Pam-Cys(Pam)-OH in CDCl ₃/CD₃OD 1:1

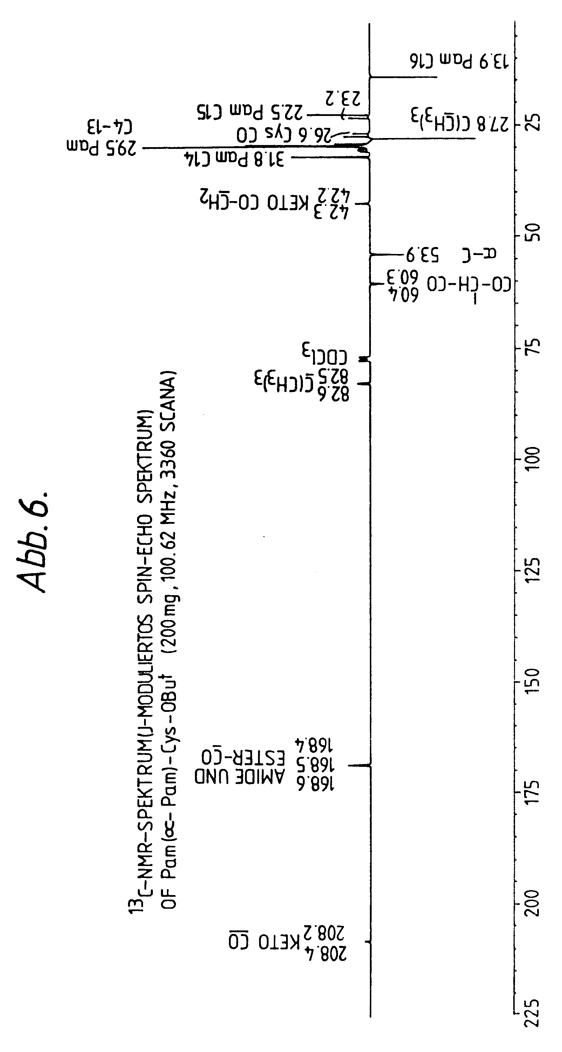

- Fig. 6: das ¹³C-NMR-Spektrum (J-moduliertes Spin-Echo-Spektrum) von Pam(α-Pam)Cys-OBu^{t})

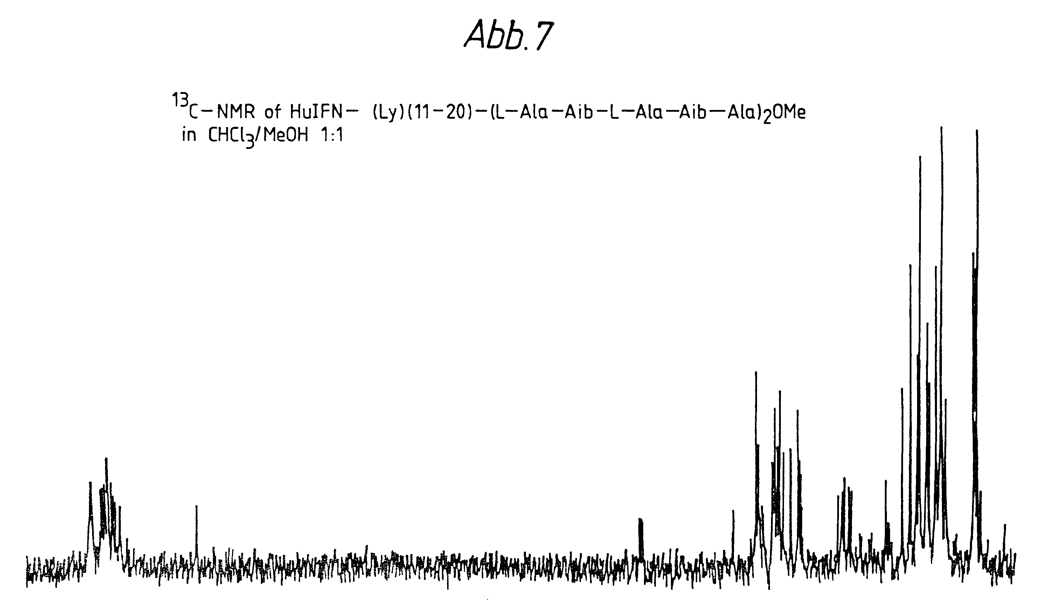

- Fig. 7: das ¹³C-NMR (100 MHz) der Alpha-Helix

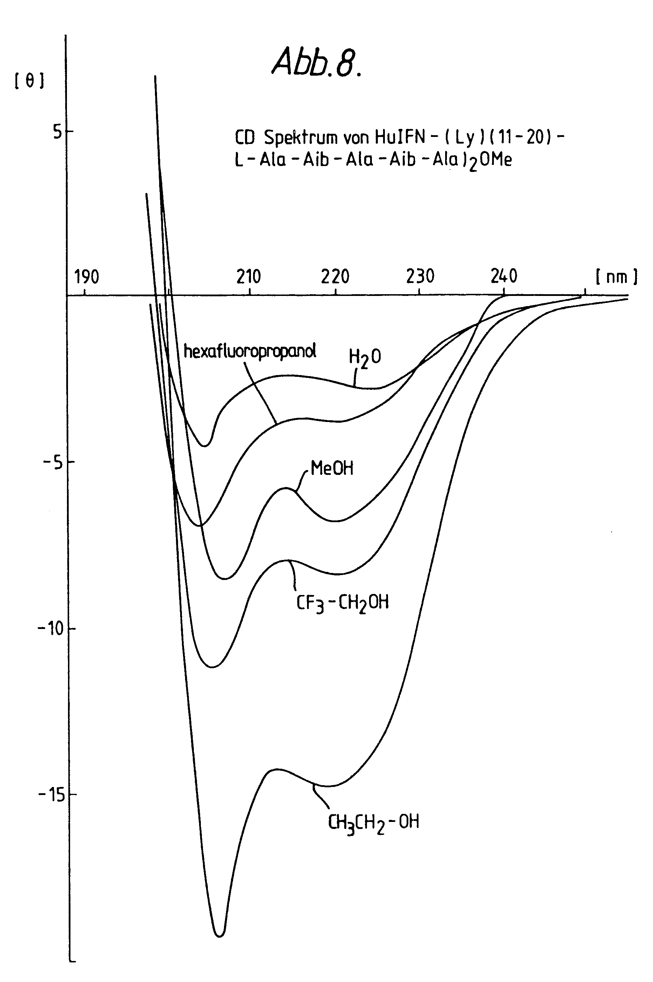

- Fig. 8: das CD-Spektrum der Alpha-Helix von HuIFN(α-Ly)-11-20
- Fig. 9: die Antikörpergewinnung mit Pam₃Cys-Ser-EGF-R (516 bis 529)
- Fig. 10: ein in vivo Immunisierungsexperiment
- Fig. 11: einen Vergleich von in vivo/in vitro Immunisierungsexperiment, und
- Fig. 12: die mitogene Aktivierung von Balb/c-Maus-Milzzellen mit Pam₃Cys-Ser-(Lys)₄FITC.

Im folgenden werden nun zunächst einige Herstellungsverfahren für erfindungsgemäße Stoffe sowie ihre Vorstufen beschrieben:

### I. Herstellung von Pam₃Cys-EGF-R(516 - 529)

Nach üblichem schrittweisen Aufbau (Merrifield-Synthese unter N α-Fmoc/(Bu^{t})-Schutz, DCC/HOBt und symmetrische Anhydride) des EGF-R Segments (526-529) wurde als letzte Aminosäure Fmoc-Ser(Bu^{t})-OH angebaut. Nach Abspaltung der Fmoc-Gruppe mit Piperidin/DMF (1:1, 15 min) wurde das harzgebundene Pentadekapeptid des EGF-RH:Ser(Bu^{t})-Asn-Leu-Leu-Glu-(0Bu^{t})-Gly-Glu(OBu^{t})-Pro-Arg(H⁺)-Glu(0Bu^{t})-Phe-Val -Glu(0Bu^{t})-Asn-Ser-(Bu^{t})-O-p-Alkoxy-benzyl-Copoly(Divinylbenzol-Styrol) (1g, Beladung 0,5 mmol/g) mit Pam-Cys (CH₂-CH(0Pam)CH₂(0Pam) (2mmol, in DMF/CH₂Cl₂ (1:1)) und DCC/HOBt (2mmol, bei 0 Grad C 20 min voraktiviert) verknüpft (16 h), dann eine Nachkupplung (4 h). Das Lipohexadekapeptid wurde mit Trifluoressigsäure (5 ml) unter Zusatz von Thioanisol (0,25 ml) innerhalb von 2 h abgespalten.

### Ausbeute:

960 mg = (76%) Pam-Cys(CH₂-CH(0Pam)CH₂(0Pam))Ser-Asn-Leu--Leu-Glu-Gly-Glu-Pro-Arg-Glu-Phe-Val-Glu-AsnSer-OH x CF₃COOH (korrekte Aminosäurenanalyse, keine Racemisierung).

### II. Herstellung von S-(1,2-Dioctadecyloxycarbonylethyl-) N-palmitoyl-L(oder D)cystein-tert-butylester

Maleinsäure-di-octadecylester ist nach der allgemeinen Vorschrift für Veresterungen von Maleinsäure zugänglich (H. Klostergaard, J. Org. Chem. 23 (1958), 108.)

### ¹³C-NMR-Spektrum:

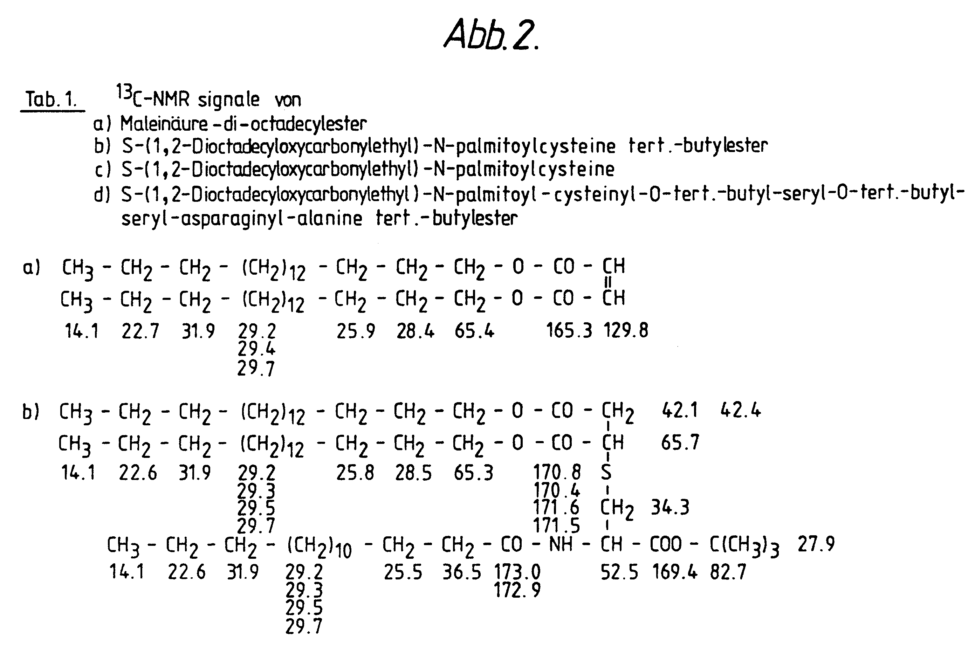

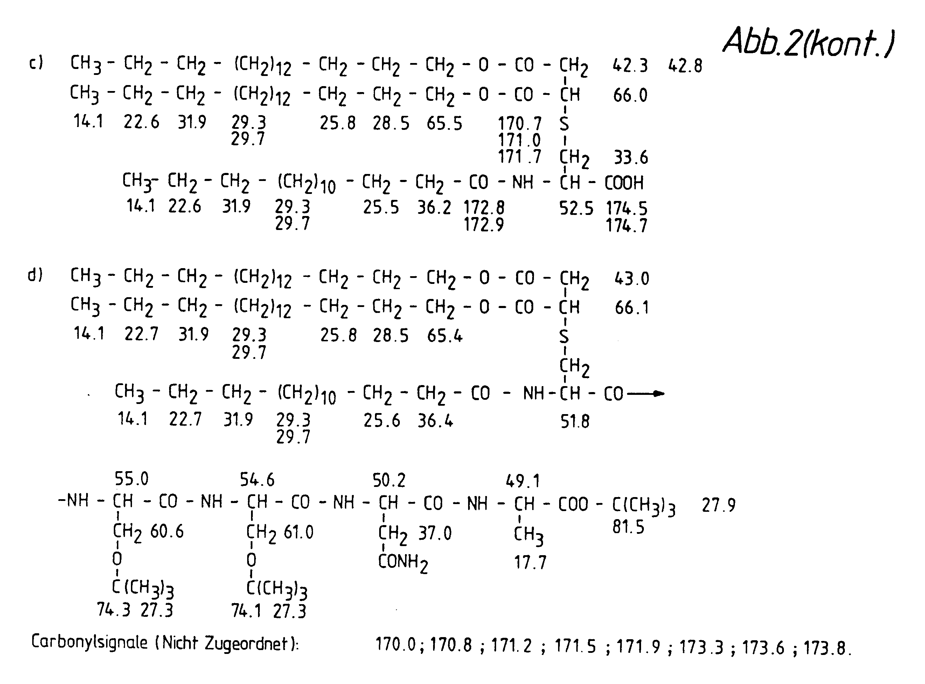

siehe Fig. 2.

1,2 mmol (500 mg) N-Palmitoyl-L-cystein-tert-butylester und 1,2 mmol (745 mg) Maleinsäure-di-octadecylester werden in 20 ml THF gelöst. Nach Zugabe von 20 mmol (3 ml) N,N,N',N'-Tetramethylethylendiamin wird unter Stickstoff am Rückflußkühler 12 h gerührt. Nach Zugabe von 100 ml Methanol und 5 ml Wasser wird der farblose Niederschlag abgesaugt, mit Wasser und Methanol gewaschen und im Vakuum über P₂O₅ getrocknet.

### Ausbeute:

1 g (83%);

### Schmelzpunkt:

51 Grad Celsius

### Dünnschichtchromatographie:

R_{F} = 0,80; (Laufmittel: CHCl₃/Essigester = 14:1)

### ¹³C-NMR:

siehe Fig. 2.

### Molekulargewicht:

C₆₃H₁₁₃NO₇S (1035,7)

### Elementaranalyse:

| | | | | |
|---|---|---|---|---|
| Ber. | C 72,99 | H 11,76 | N 1,35 | S 3,09 |
| Gef. | C 73,08 | H 11,92 | N 1,27 | S 3,27 |

### III. Herstellung von S-(1,2-Dioctadecyloxycarbonylethyl) N-palmitoyl-cystein

0,48 mmol (500 mg) des unter II. beschriebenen t-Butylesters werden in 65,3 mmol (7,45 g, 5 ml) Trifluoressigsäure 1 h bei Raumtemperatur im geschlossenen Gefäß gerührt. Es wird am Rotationsverdampfer im Hochvakuum abgedampft, der Rückstand in 1 ml Chloroform aufgenommen, mit 50 ml Petrolether bei -20 Grad C ausgefällt und über P₂O₅ im Vakuum getrocknet.

### Ausbeute:

420 mg (89%);

### Schmelzpunkt:

64 Grad Celsius

### Dünnschichtchromatographie auf Silicagelplatten:

R_{F} = 0,73; Laufmittel: CHCl₃/MeOH/H₂O = 65:25:4)

### ¹³C-NMR:

siehe Tab. 1.

### Molekulargewicht:

C₅₉H₁₁₃NO₇S (980,6)

### Elementaranalyse:

| | | | | |
|---|---|---|---|---|
| Ber. | C 72,27 | H 11,62 | N 1,43 | S 3,27 |
| Gef. | C 72,46 | H 11,75 | N 1,36 | S 3,50 |

Das neue Cysteinderivat und sein t-Butylester lassen sich an Kieselgel und RP-Phasen-Chromatographie in die Diastereomeren trennen. Es können so die beiden Diastereomerenpaare des L- und D- Cysteinderivats hergestellt werden.

### IV. Herstellung von S-(1,2-Dioctadecyloxycarbonylethyl)-N-palmitoyl-Cys-Ser(Bu^{t})-Ser(Bu^{t})-Asn-Ala-OBu^{t}

0,2 mmol (196 mg) S-(1,2-Dioctedecyloxycarbonylethyl)-N-palmitoyl-cystein werden in 5 ml Dichlormethan gelöst und mit 0,2 mmol (27 mg) HOBt in 0,5 ml DMF sowie 0,2 mmol (41 mg) DCC 30 min bei 0 Grad C unter Rühren voraktiviert.

Nach Zugabe von 0,2 mmol (109 mg) H-Ser(Bu^{t})-Ser(Bu^{t})-Asn-Ala-OBu^{t} in 3 ml Dichlormethan wird 12 Stunden bei Raumtemperatur gerührt. Ohne weitere Aufarbeitung werden 40 ml Methanol zum Reaktionsansatz gegeben.Nach 3 h läßt sich das farblose Produkt absaugen. Es wird in wenig Dichlormethan aufgenommen und erneut mit Methanol gefällt. Nach Waschen mit Methanol wird im Vakuum über P₂O₅ getrocknet.

### Ausbeute:

260 mg (86%)

### Schmelzpunkt:

194 Grad Celsius

### Dünnschichtchromatographie:

R_{F} = 0,95; (Laufmittel: CHCl₃/MeOH/H₂O = 65:25:4)
R_{F} = 0,70; (Laufmittel: CHCl₃/MeOH/Eisessig = 90:10:1)

### ¹³C-NMR:

siehe Fig. 2

### Molekulargewicht:

C₈₄H₁₅₈N₆O₁₄S (1508,3)

### Elementaranalyse:

| | | | |
|---|---|---|---|
| Ber. | C 66,89 | H 10,56 | N 5,57 |
| Gef. | C 67,10 | H 10,41 | N 5,52 |

### V. Herstellung von S-(1,2-Dioctadecyloxycarbonylethyl)-N-Palmitoyl-Cys-Ser-Ser-Asn-Ala

53 »mol (80 mg) geschützes Lipopeptid (IV) werden mit 13 mmol (1,5 g; 1 ml) Trifluoressigsäure 1 h bei Raumtemperatur im geschlossenen Gefäß gerührt. Nach Abdampfen im Hochvakuum wird 2 x mit je 10 ml Dichlormethan aufgenommen und jeweils am Rotationsverdampfer abgedampft. Der Rückstand wird in 3 ml Chloroform aufgenommen und mit 5 ml Methanol bei 4 Grad Celsius innerhalb von 12 h gefällt. Nach dem Absaugen wird mit Methanol gewaschen und im Exsikkator über P₂O₅ getrocknet.

### Ausbeute:

63mg (87%)

### Schmelzpunkt:

208 Grad Celsius (Zers.)

### Dünnschichtchromatographie:

R_{F} = 0,63; (Laufmittel: CHCl₃/MeOH/Eisessig/H₂O = 64:25:3:4)
R_{F} = 0,55; (Laufmittel: CHCl₃/MeOH/H₂O = 64:25:4)
R_{F} = 0,06; (Laufmittel: CHCl₃/MeOH/Eisessig = 90:10:1)

### Aminosäurenanalyse:

Cysteinsäure 0,6; Asparaginsäure 0,93; Serin 1,8; Alanin 1,0

### Molekulargewicht:

C₇₂H₁₃₄N₆O₁₄S (1340)

### VI. Herstellung von Pam₃Cys-Ser-(Lys)₄-OH:

Pam₃Cys-Ser(Lys)₄-OH wurde nach der Festphasenmethode (MERRIFIELD) an einem p-Alkoxybenzylalkohol-PS-DVB(1%)Copolymer mit N-Fmoc-Aminosäuren und säurelabilem Seitenkettenschutz (tBu für Serin und Boc für Lysin) aufgebaut. Es wurden die symmetrischen Anhydride der Fmoc-Aminosäuren eingesetzt. Die Kupplung an Pam₃Cys-OH wurde nach der DCC/HOBt-Methode durchgeführt und wiederholt, um eine möglichst quantitative Umsetzung zu erreichen. Um das Lipopeptid vom Trägerharz abzuspalten und den Seitenkettenschutz zu entfernen, wurde das Harz zweimal 1,5 h mit Trifluoressigsäure behandelt, die anschließend am Rotationsverdampfer im Hochvakuum entfernt wurde. Das Produkt wurde aus Aceton umkristallisiert.

Die Elementaranalyse weist ebenso wie das ¹³C-Spektrum darauf hin, daß das Lipopeptid als Trifluoracetat vorliegt. Unter der Annahme, daß Pam₃Cys-Ser-(Lys)₄-OH als Zwitterion vorliegt, bleiben noch drei ε-Aminogruppen übrig, die von drei Trifluoressigsäure-Molekülen protoniert werden können.

Das ¹³C-NMR-Spektrum von Pam₃Cys-Ser-(Lys)₄-OH x 3 TFE zeigt, daß die Verbindung als Trifluoracetat vorliegt. (Quartett der CF₃-Gruppierung bei 110-120 ppm, sowie Carbonylsignale bei 161-162 ppm). Durch die Aggregation des polaren Molekülteils werden die Linien des Lys- und Ser-C-Atome stark verbreitert. Das Carbonyl-Signal bei 206,9 ppm rührt von noch anhaftendem Aceton, das zur Umkristallisation verwendet wurde, her.

### Molekulargewicht:

1510,4

### Elementaranalyse:

| | | | | |
|---|---|---|---|---|
| Ber. | C 56,40 | H 8,70 | N 7,56 | S 1,73 |
| Gef. | C 55,58 | H 9,33 | N 6,54 | S 2,61 |

### Aminosäurenanalyse:

Die Aminosäureanalyse ergab ein Verhältnis von Serin zu Lysin wie 1 : 4,2. Die bei der Hydrolyse (6 N HCl, 110 °C, 18h) entstehenden charakteristischen Zersetzungsprodukte von S-Glyceryl-cystein waren vorhanden (Vergleich mit bekannten Standards). Die peptidische Anteil wurde zu 83% berechnet. 3 TFE-Moleküle pro Lipopeptid entsprechen einem peptidischen Anteil von 80,2 % in guter Übereinstimmung mit der Analyse.

### VII. Herstellung von Pam₃Cys-Ser-(Lys)₄-OH x 3 TFE

### VII.1. Kupplung von Fmoc-Lys(Boc)-OH an das Trägerharz

Fmoc-Lys(Boc)-OH (4,5 g, 9,6 mmol) wird in 15 bis 20 ml DMF/CH₂Cl₂ 1:1 (v/v) bei 0 Grad C mit DCC (0,99 g, 4,8 mmol) versetzt. Nach 30 min wird von ausgefallenem Harnstoff direkt in eine Schüttelente abfiltriert, in welcher sich p-Benzyloxy-benzyl-alkohol-Harz (2,5 g, 1,6 mmol OH-Gruppen) befinden. Nach Zugabe von Pyridin (0,39 ml, 4,8 mmol) wird 18 h bei Raumtemperatur geschüttelt. Das Lösungsmittel wird abgesaugt und das Harz je 3 x mit je 20 ml DMF/CH₂Cl₂ und DMF gewaschen. Das Harz wird in 20 ml CH₂Cl₂ zuerst mit Pyridin (28,8 mmol, 6 Äq) und dann mit Benzoylchlorid (28,8 mmol, 6 Äq) versetzt. Es wird 1 h bei Raumtemperatur geschüttelt. Das Lösungsmittel wird abgesaugt und das Harz je 3 x mit 20 ml CH₂Cl₂, DMF, Isopropanol und PE-30/50 gewaschen.

### VII.2. Symmetrisches Fmoc-Aminosäureanhydrid

Fmoc-Lys(Boc)-OH (4,5 g, 9,6 mmol, 3 Äq) wird in 15 ml CH₂Cl₂/DMF gelöst und bei 0 Grad Celsius mit DCC(4,8 mmol, 1,5 Äq) versetzt. Nach 30 min bei 0 Grad Celsius wird vom Harnstoff direkt in den Reaktor abfiltriert und, wie in nachfolgender Tabelle angegeben, weiterverarbeitet.

Die folgende Vorschrift gilt für 1/5 der zu Anfang eingesetzten Harzmenge (0,5 g, 0,32 mmol OH-Gruppen).

Fmoc-0-Butyl-Serin (0,74 g, 1,92 mmol) wird in 4 ml CH₂Cl₂/DMF gelöst und bei 0 Grad Celsius mit DCC (0,96 mmol) versetzt.

**Tabelle**

| Sequenzaufbau eines Peptids mit symmetrischen Fmoc-Aminosäureanhydriden | | | |
|---|---|---|---|
| Operation | Reagenz | Zeit[min] | Häufigkeit |
| 1 | CH₂Cl₂ | 2 | 2 |
| 2 | DMF | 2 | 2 |
| 3 | 55 % Piperidin /DMF (v/v) | 5 | 1 |
| 4 | 55 % Piperidin /DMF (v/v) | 10 | 1 |
| 5 | DMF | 2 | 3 |
| 6 | Isopropanol | 5 | 2 |
| 7 | DMF | 2 | 3 |
| 8 | CH₂Cl₂ | 2 | 3 |
| 9 | DMF | 2 | 2 |
| 10 | Kupplung mit 3 Äq. symmetr. Fmoc-Aminosäure-anhydrid in DMF/CH₂Cl₂ 1:1 (v:v); nach 15 min Zugabe von 3 Äq. NMM | 90 | 1 |
| 11 | DMF | 2 | 3 |
| 12 | CH₂Cl₂ | 2 | 3 |
| 13 | Überprüfung der Vollständigkeit der Kupplung mittels Kaiser-Test ;wenn nötig Schritte 10-12 wiederholen | | |
| 14 | Acetylieren: 2 Äq. Ac₂O und 0.5 Äq. NMM in 20 ml CH₂Cl₂ | 15 | 1 |
| 15 | CH₂Cl₂ | 2 | 3 |
| 16 | Isopropanol | 2 | 3 |
| 17 | CH₂Cl₂ | 2 | 3 |

Nach 30 min wird bei 0 Grad C vom Harnstoff direkt in den Reaktor abfiltriert und, wie üblich aufgearbeitet.

### VII.3. Kupplung an Pam₃Cys-OH

Pam₃Cys-OH (0,58 g, 0,64 mmol) wird in 5 ml CH₂Cl₂/DMF 1:1 (v:v) gelöst und bei 0 °C mit HOBt (93 mg, 0,64 mmol) und DCC (0,64 mmol) versetzt. Nach 30 min bei 0 °C wird das Gemisch direkt in den Reaktor gegeben. Nach 16 h Schütteln wird mit den gleichen Molverhältnissen wie oben 4 h lang nachgekuppelt. Das Lösungsmittel wird abgesaugt und das Harz je 3 x mit 20 ml DMF/CH₂Cl₂ und DMF gewaschen.

### VII.4. Abspaltung des Hexapeptids vom Polymer

Die Boc-geschützte Peptid-Polymerharz-Verbindung (ca. 1 g) aus VII.3 wird gut mit CH₂Cl₂ gewaschen und 2 x 1,5 h mit einer Mischung aus 5 ml TFE und 0,5 ml Anisol geschüttelt. Das Filtrat wird im Vakuum eingeengt und der Rückstand in 5 ml CHCl₃ aufgenommen. Pam₃Cys-Ser-(Lys)₄-OH x 3 TFE kristallisiert nach Zugabe von 50 ml Aceton bei -20 Grad Celsius aus, wird abzentrifugiert und im Hochvakuum getrocknet.

### Ausbeute:

0,41 g (85%)

### Schmelzpunkt:

205 Grad Celsius (Zers.)

### Dünnschichtchromatographie auf Silikagelplatten:

R_{F} = 0,42;
(Laufmittel: n-BuOH/Pyridin/H₂0/Eisessig = 4:1:1:2)
R_{F} = 0,82
(Laufmittel: n-BuOH/MeOH/H₂0/Eisessig = 10:4:10:6)

### Aminosäurenanalyse:

Ser 0,95 (1); Lys 4 (4)

### Molekulargewicht:

C₈₇H₁₅₉N₁₀O₁₉SF₉ (1852,6)

### Elementaranalyse:

| | | | | |
|---|---|---|---|---|
| Ber. | C 56,40 | H 8,70 | N 7,56 | S 1,73 |
| Gef. | C 55,58 | H 9,33 | N 6,94 | S 2,61 |

### VIII. Herstellung von Pam₃Cys-Ser-(Lys)₄-OH-FITC x 2 TFE

Fluoresceinisothiocyanat (3,9 mg, 10 Mikromol) wird in 2 ml Chloroform gelöst und zu einer Lösung von Pam₃Cys-Ser-(Lys)₄-OH x 3 TFE (18,5 mg, 10 Mikromol) in 2 ml Chloroform gegeben. Nach Zugabe von 4-Methylmorpholin (10 Mikroliter, 10 Mikromol) wird 1 h gerührt und das Lösungsmittel anschließend am Rotationsverdampfer entfernt. Der Rückstand wird in 10 ml Chloroform/Aceton 1:1 gelöst. Das gelbe Produkt fällt bei -20 Grad Celsius voluminös aus und wird abzentrifugiert und im Hochvakuum getrocknet.

### Ausbeute:

### 16 mg nach Reinigung an Sephadex LH20

Das Produkt liegt als Trifluoracetat vor und fluoresziert sehr stark bei Anregung mit UV-Licht der Wellenlänge 366 nm. Im Vergleich zum Ausgangsprodukt ist eine ε-Aminogruppe kovalent mit FITC verknüpft. Daraus resultiert die Summenformel Pam₃Cys-Ser-(Lys)₄-OH-FITC x 2 TFE, vorausgesetzt die zwitterionische Struktur bleibt erhalten.

### Molekulargewicht:

C₁₀₆H₁₆₉N₁₁O₂₂S₂F₆ (2127,68)

### Dünnschichtchromatographie auf Silikagelplatten:

R_{F} = 0,72
(Laufmittel: n-Butanol/Pyridin/Wasser/Eisessig = 4:1:1:2)
R_{F} = 0,73
(Laufmittel: n-Butanol/Ameisensäure/Wasser = 7:4:2)

### Aminosäurenanalyse:

Ser 1,11 (1,00) Lys 4,00 (4,00)
Die Hydrolyseprodukte von Glycerylcystein sind vorhanden.

### IX. Herstellung von Pam₃Cys-Ser-(Lys)₄-OH x 3HCl

Pam₃Cys-Ser-(Lys)₄-OH x 3 TFE (185,2 mg, 0,1 mmol) werden in wenig Chloroform gerade gelöst und etwa das gleiche Volumen etherische HCl-Lösung zugegeben. Es wird gut geschüttelt, wobei ein Teil ausfällt, der größte Teil jedoch in Lösung bleibt. Es wird am Rotationsverdampfer bis zur Trockene einrotiert und erneut Ether/HCl zugegeben. Nach mehrfacher Wiederholung dieses Vorgangs wird in wenig Chloroform der Rückstand gelöst und bis zum Trübwerden der Lösung Aceton zugegeben. Das Produkt kristallisiert als farbloses Pulver bei -20 Grad C aus und wird abgesaugt und im Hochvakuum getrocknet.

### Ausbeute:

153 mg

### Molekulargewicht:

C₈₁H₁₅₉N₁₀O₁₃SCl₃ (1619, 63)

### Elementaranalyse:

| | | | |
|---|---|---|---|
| Ber. | C 60,07 | H 9,89 | N 8,65 |
| Gef. | C 57,64 | H 11,20 | N 8,39 |

Dem Produkt haftet noch überschüssige HCl an.

### Felddesorptions-Massenspektrometrie:

Der M⁺-Peak erscheint bei m/e 1510, sowie M⁺+1 und M⁺+2. Charakteristisch sind die protonierten Fragmente Pam₃Cys-NH (908,5) bei m/e 909, 910, 911 und 912.

### X. N,S-Dipalmitoyl-cystein-tert.butylester

Palmitinsäure (2,5 g, 9,6 mmol), Dimethylaminopyridin (130 mg, 0,9 mmol) und Dicyclohexylcarbodiimid (9,6 mmol) werden in 100 ml Chloroform gelöst. Die Lösung wird eine halbe Stunde gerührt und N-Palmitoyl-cystein-tert.butylester (2 g, 4,8 mmol), der zuvor in 50 ml Chloroform gelöst wurde, zur anderen Lösung zugetropft. Nach 1 1/2 Stunden wird das Lösungsmittel am Rotationsverdampfer entfernt und der Rückstand in 100 ml Chloroform/Methanol 1:5 aufgenommen. Das Produkt fällt bei -20 Grad C voluminös aus. Es wird abgesaugt und im Hochvakuum getrocknet.

### Ausbeute:

2,3 g (73%)

### Molekulargewicht: (Massenspektrometer)

C₃₉H₇₅NO₄S (655,20)

### Elementaranalyse:

| | | | | |
|---|---|---|---|---|
| Ber.: | C 71,48 | H 11,71 | N 2,13 | S 4,89 |
| Gef.: | C 71,72 | H 12,14 | N 2,12 | S 4,77 |

### Dünnschichtchromatographie auf Silicagelplatten:

R_{F} = 0,67 (Laufmittel: Chloroform/Essigester 95:5)
R_{F} = 0,73 (Laufmittel: Chloroform/Cyclohexan/MeOH 10:7:1)

### ¹³C-NMR:

s.Figur 4

### XI. N-(α-Tetradecyl-β-hydroxy-octadecanoyl)-cystein-tert.butylester

N-(α-Palmitoyl-palmitoyl)-cystein-tert.butylester (1,5 g 2,3 mmol) wird in 10 ml i-Propanol gelöst und die 1,5-fache molare Menge Natriumborhydrid zugegeben. Es wird zwei Stunden gerührt und nach beendeter Reaktion stickstoffgesättigte 1N Salzsäure zugegeben, bis keine Wasserstoffentwicklung mehr erfolgt. Dabei fällt das Produkt voluminös aus. Es wird abgesaugt, mehrfach mit stickstoffgesättigtem Wasser gewaschen und im Hochvakuum getrocknet.

### Ausbeute:

1,4 g (93%)

### Molekulargewicht: (bestimmt aus Massenspektrum)

C₃₉H₇₇NO₄S = 656,11

### Dünnschichtchromatographie auf Silicalgelplatten:

R_{F} = 0,84 (Laufmittel: Chloroform/Essigester 95:5)

### Elementaranalyse:

| | | | | |
|---|---|---|---|---|
| Ber.: | C 71,39 | H 11,83 | N 2,13 | S 4,89 |
| Gef.: | C 71,32 | H 12,39 | N 2,04 | S 5,33 |

### XII. N-(α-Tetradecyl-β-hydroxy-octadecanoyl)-cystein

N-(α-Tetradecyl-β-hydroxy-octadecanoyl)-cystein-tert.butyl-ester (1g, 1,5 mmol) wird 1/2 Stunde mit wasserfreier Trifluoressigsäure behandelt und diese anschließend im Hochvakuum am Rotationsverdampfer entfernt. Der Rückstand wird in tert.Butanol gelöst und lyophilisiert.

### Ausbeute:

0,7 g (78%)

### Molekulargewicht: (bestimmt aus Massenspektrum)

C₃₅H₆₉NO₄S 600,0

### Elementaranalyse:

| | | | | |
|---|---|---|---|---|
| Ber.: | C 70,06 | H 10,92 | N 2,33 | S 5,34 |
| Gef.: | C 70,36 | H 10,44 | N 2,45 | S 5,01 |

### Dünnschichtchromatographie auf Silicalgelplatte:

R_{F} = 0,43 (Laufmittel:Chloroform/Methanol/Wasser 65:25:4)

### XIII. N,S-Dipalmitoyl-cystein

N,S-Dipalmitoyl-cystein-tert.butylester (1 g, 1,5 mmol) wird 1 h lang mit wasserfreier Trifluoressigsäure behandelt. Anschließend wird diese am Rotationsverdampfer im Hochvakuum entfernt, der Rückstand in tert. Butanol aufgenommen und lyophilisiert.

### Ausbeute:

0,8 g (89%)

### Molekulargewicht: (bestimmt aus Massenspektrum)

C₃₅H₆₇NO₄S (598,00)

### Elementaranalyse:

| | | | | |
|---|---|---|---|---|
| Ber.: | C 70,18 | H 11,44 | N 2,34 | S 5,34 |
| Gef.: | C 69,97 | H 11,31 | N 2,50 | S 5,17 |

### Dünnschichtchromatographie:

R_{F} = 0,30
(Laufmittel: Chloroform/Methanol/Eisessig 90:10:1)
R_{F} = 0,75
(Laufmittel: Chloroform/Methanol/Wasser 65:25:4)
R_{F} = 0,81
(Laufmittel: Chloroform/Methanol/Ammoniak(25%)/Wasser 65:25:3:4)

### ¹³C-NMR:

s. Fig. 5

### XIV. N-(α-Palmitoyl-palmitoyl)-N'-palmitoyl-cystein-di-tert.butylester

Palmitoylchlorid (8g, 30 mmol) wird in 40 ml stickstoffgesättigtem Dimethylformamid gelöst und Triethylamin (60 ml, 60 mmol) zugegeben. Die Mischung wird drei Stunden im Stickstoffstrom unter Rückfluß gekocht, wobei das bei der Bildung des Tetradecylketen Dimeren entstehende Triethylammoniumchlorid als farbloses Salz ausfällt. Der Ruckflußkühler wird anschließend durch einen Tropftrichter ersetzt und eine Lösung von Cystein-di-tert.butylester (4,9 g, 15 mmol) in 20 ml Dimethylformamid langsam zugetropft. Nach 6 Stunden wird das Lösungsmittel am Rotationsverdampfer entfernt, der Rückstand in Chloroform aufgenommen und je zweimal mit je 100 ml 5%-iger Kaliumhydrogensulfatlösung und einmal mit 1200 ml Wasser gewaschen. Die organische Phase wird über wasserfreiem Natriumsulfat getrocknet und das Lösungsmittel erneut entfernt. Bei - 20 Grad Celsius kristallisiert ein Gemisch aus N-(α-Palmitoyl-palmitoyl)-N'-palmitoyl-cystein-tert.butyl-ester und N,N'-Dipalmitoyl-cystein-di-tert.butylester aus, das durch Gelfiltration an Sephadex LH-20 in Choloroform/Methanol 1:1 getrennt wird.

### Ausbeute:

6,4 g (40%)

### Molekulargewicht:(Massenspektrum)

C₆₂H₁₁₈N₂O₇S (1067,76)

### Dünnschichtchromatographie auf Silikagelplatten:

R_{F} = 0,69 (Laufmittel: Chloroform/-Essigester 91:5)

### XV. N-(α-Palmitoyl-palmitoyl)-cystein-tert.butylester

N-(α-Palmitoyl-palmitoyl)-N'-palmitoyl-cystein-di-tert.-butylester (3,2 g, 3 mmol) wird in wenig Methylenchlorid gelöst und 100 ml 9,1 N methanolische Salzsäure zugegeben. Die Lösung wird in eine Elektrolysezelle mit einer Silberelektrode als Anode und Quecksilber als Kathode überführt und bei einer konstanten. Spannung von -1,1 V reduziert. Die Stromstärke fällt am Ende der elektrochemischen Reduktion von etwa 200 mA auf fast Null ab. Das Lösungsmittel wird anschließend am Rotationsverdampfer entfernt und das Produktgemisch aus N-(α-Palmitoyl-palmitoyl)-cystein-tert.butylester und N-Palmitoyl-cystein-tert.butylester aus Methanol bei -20 Grad Celsius gefällt. Diese beiden Verbindungen werden durch Gelfiltration an Sephadex LH-20 in Chloroform/Methanol 1:1 getrennt.

### Ausbeute :

1,5 g (76%)

### Molekulargewicht: (bestimmt aus Massenspektrum)

C₃₉H₇₅NO₄S 654,09

### Dünnschichtchromatographie auf Silicalgelplatten:

R_{F} = 0,75 (Laufmittel: Chloroform/Essigester 95:5)

### Elementaranalyse:

| | | | | |
|---|---|---|---|---|
| Ber.: | C 71,48 | H 11,71 | N 2,13 | S 4,89 |
| Gef.: | C 71,16 | H 11,31 | N 2,00 | S 4,65 |

### XVI. Herstellung eines Antigen-Konjugats mit einem konformationsstabilisierten α-helikalen Membrananker

Synthese von HuIFN-α(Ly)(11-20)-(L-Ala-Aib-Ala-Aib-Ala)2-OMe, ein 20-Peptid, das N-terminal eine antigene Determinante von menschlichem Interferon (α(Ly)) trägt.

Die Synthese des lipophilen Membranankers mit funktioneller Amino-Kopfgruppe H-(Ala-Aib-Ala-Aib-Ala)2-OMe kann für andere Konjugate übernommen werden. Die Alpha-Helix kann auch ein- oder zweimal um die Einheit Ala-Aib-Ala-Aib-Ala verlängert werden. Dazu wird vorteilhaft vom Pentapeptid Boc-Ala-Aib-L-Ala-Aib-L-Ala-OMe ausgegangen. (R. Oekonomopulos, G. Jung, Liebigs Ann. Chem. 1979, 1151; H. Schmitt, W. Winter, R. Bosch, G. Jung, Liebigs Ann..Chem. 1982 , 1304).

### XVII. Herstellung von Boc-Asn-Arg(NO₂)-Arg(NO₂)-OH

### XVII.1. Boc-Arg(NO₂)-OMe

Boc-Arg(NO₂)-OMe (15.97 g, 50 mmol) und HOBt (6.67 g, 50 mmol) in DMF (100 ml) wurden bei -10 Grad Celsius zu HCl x H-Arg(NO₂)-OMe (13.49 g, 50 mmol) und NMM (5.5 ml, 50 mmol) in CH₂Cl₂ (12 ml) bei - 10 Grad C gegeben und die Mischung wurde 30 min bei - 10 Grad Celsius, 1 Stunde bei 0 Grad Celsius und 3 Stunden bei Raumtemperatur gerührt. Danach wurde die Reaktion mit einigen Tropfen Eisessig abgestoppt. Es wurde vom ausgefallenen DCH abfiltriert und das Lösungsmittel am Hochvakuum abgezogen. Der ölige Rückstand wird in Essigester unter Zusatz von etwas n-Butanol gelöst. Nach Waschen mit 5% KHSO₄-, 5% KHCO₃- und gesättigter NaCl-Lösung wurde die organische Phase über Na₂SO₄ getrocknet, mit Petrolether (30-50) versetzt und in der Kälte gefällt.

### Ausbeute:

20.30 g (76%);

### Schmelzpunkt:

130 Grad Celsius (Zersetzung);

### Dünnschichtchromatographie:

R_{F}(I) = 0.69,
R_{F}(II) = 0.87,
R_{F}(III) = 0.81,
R_{F}(IV) = 0.32,
R_{F}(V) = 0.42.

### Molekulargewichtsbestimmung

C₁₈H₅₄N₁₀O₉ (534.5)

### Elementaranalyse:

| | | | |
|---|---|---|---|
| ber. | C 40.45 | H 6.41 | N 26.20 |
| gef. | C.40.39 | H 6.55 | N 26.11 |

### XVII.2. HCl x H-Arg(NO₂)-Arg(NO₂)-OMe

Boc-Arg-(NO₂)-Arg(NO₂)-OMe (20.00 g, 37.42 mmol) wurde mit 1.2 N HCl/Essigsäure (110 ml) versetzt und nach 30 min in gerührten Ether (600 ml) gegossen. Dabei fiel dünnschichtchromatographisch reines HCl x H-Arg(NO₂)-Arg(NO₂)-OMe aus.

### Ausbeute:

17.3 g (98%);

### Dünnschichtchromatographie auf Silikagelplatten:

R_{F}(I) = 0.37,
R_{F}(II) = 0.29,
R_{F}(III) = 0.44,
R_{F}(IV) = 0.07,
R_{F}(V) = 0.10.

### XVII.3. Boc-Asn-Arg(NO₂)-Arg(NO₂)-OMe

Boc-Asn-OH (8.39 g, 36.10 mmol) und HOBt (4.89 g, 36.10 mmol) in DMF (75 ml) wurden bei - 10 °C zu HCl x H-Arg(NO₂)-Arg(NO₂)-OMe (17.00 g, 36.10 mmol) und NMM (3.98 mmol) in DMF (75 ml) gegeben. Nach Zugabe von DCC (7.53 g, 36.50 mmol) in CH₂Cl₂ (10 ml) wurde 30 min bei -10 Grad Celsius, 1 Stunde bei 0 Grad Celsius und 3 Stunden bei Raumtemperatur gerührt. Nach Abbruch der Reaktion mit wenigen Tropfen Eisessig wird das Lösungsmittel im Vakuum verdampft und der Rückstand in wenig Methanol aufgenommen. Diese Lösung wird unter Rühren zu trockenem Ether getropft. Der Rückstand wird abfiltriert und in Methanol aufgenommen. In der Kälte fällt reines Produkt aus.

### Ausbeute:

18.25g (78%)

### Schmelzpunkt:

170 Grad Celsius

### Dünnschichtchromatographie

R_{F}(I) = 0.59,
R_{F}(II) = 0.67,
R_{F}(III) = 0.66,
R_{F}(IV) = 0.45,
R_{F}(V) = 0.65.

### Aminosäurenanalyse:

Asx 1.00 (1), Arg 1.85 (2)

### Molekulargewichtsbestimmung:

C₂₂H₄₀N₁₂O₁₁ (648.6)

### Elementaranalyse:

| | | | |
|---|---|---|---|
| ber. | C 40.74 | H 6.22 | N 25.91 |
| gef. | C 40.70 | H 6.40 | N 25.79 |

### XVII.4. Boc-Asn-Arg(NO₂)-Arg(NO₂)-OH

Boc-Asn-Arg(NO₂)-Arg(NO₂)-OMe (18.00 g, 27.75 mmol) wurde in Methanol (180 ml) mit 1 N NaOH (80 ml) bei Raumtemperatur verseift. Nach 2 h wurde mit verdünnter HCl neutralisiert und das Methanol im Vakuum abgedampft. Bei pH 3 wurde erschöpfend mit Essigester extrahiert. Die organischen Phasen wurden mit wenig gesättigter NaCl-Lösung gewaschen, über Na₂SO₄ getrocknet und aus einer methanolischen Lösung bei -20 Grad Celsius kristallisiert.

### Ausbeute:

15.84 g (90%);

### Schmelzpunkt:

228 Grad Celsius (Zersetzung)

### Dünnschichtchromatographie

R_{F}(I) = = 0.49,
R_{F}(II) = 0.21
R_{F}(III) = 0.26
R_{F}(IV) = 0.05,
R_{F}(V) = 0.21

### XVIII. Boc-Ala-Leu-Ile-Leu-Leu-Ala-Gln-(Ala-AibXAla-Aib-Ala)₂-OMe

### XVIII.1 Boc-Ala-Aib-Ala-Aib-Ala-OH

Boc-Ala-Aib-Ala-Aib-Ala-OMe (10.03 g, 20 mmol) wurde in MeOH (150 ml) mit 1 N NaOH (40 ml, 40 mmol) verseift. Nach 2.5. Stunden wurde mit 1 N HCl neutralisiert, im Vakuum eingedampft und zwischen EE/5% KHCO₃ (1:1; 1000 ml) verteilt. Die wäßrige Phase wurde mit 5% KHSO₄ auf pH 4 angesäuert und mit EE/1-Butanol (5:1) fünfmal extrahiert. Die organische Phase wurde über Na₂SO₄ getrocknet, mit PE (30-50) versetzt und die Pentapeptidsäure in der Kälte gefällt.

### Ausbeute

6.54 g (65%)

### Schmelzpunkt:

195 Grad Celsius (Zersetzung)

### Dünnschichtchromatographie

R_{F}(I) = 0.72;
R_{F}(II) = 0.80,
R_{F}(III) = 0.87,
R_{F}(IV) = 0.95,
R_{F}(V) = 0.80.

### Aminosäurenanalyse:

Ala 3.08 (3), Aib 1.98 (2).

### Molekulargewicht:

C₂₂H₃₉N₅O₈ (501.6)

### Elementaranalyse:

| | | | |
|---|---|---|---|
| ber. | C 52.68 | H 7.84 | N 13.96 |
| gef. | C 52.70 | H 7.90 | N 13.89 |

### XVIII.2. Boc-(Ala-Aib-Ala-Aib-Ala)₂-OMe

Boc-Ala-Aib-Ala-Aib-Ala-OH (1.75 g, 3.48 mmol) und HOBt (470 mg, 3.48 mmol) in DMF(10 ml) wurden bei -10 Grad Celsius zu HCl x H-Ala-Aib-Ala-Aib-Ala-OMe (1.57 g, 3.48 mmol) und NMM (384»l, 3.48 mmol) in DMF (8 ml) gegeben. Nach Zugabe von DCC (825 mg) 4.00 mmol) in CH₂Cl₂ (3 ml) bei - 10 Grad Celsius wurde unter langsamen, selbständigem Erwärmen 15 h gerührt. Nach Abstoppen der Reaktion mit einigen Tropfen Eisessig wurde vom ausgefallenen DCH abzentrifugiert, der Rückstand zweimal mit kaltem DMF gewaschen und das Lösungsmittel im Vakuum abgedampft. Der Rückstand wurde in 10 ml CHCl₃/MeOH 1:1 aufgenommen und an Sephadex LH 20 in CHCl₃/MeOH 1:1 chromatographiert.

### Ausbeute:

2.246 g (72%),

### Schmelzpunkt:

160 Grad Celsius,

### Dünnschichtchromatographie

R_{F}(I) = 0.61,
R_{F}(II) = 0.76,
R_{F}(III) = 0.83,
R_{F}(IV) = 0.95,
R_{F}(V) = 0.81.

### Molekulargewichtsbestimmung:

C₄₀H₇₀N₁₀O₁₃ (899.1)

### Elementaranalyse:

| | | | |
|---|---|---|---|
| ber. | C 53.44 | H 7.85 | N 15.58 |
| gef. | C 53.42 | H 7.90 | N 15.40 |

### XVIII.3. HCl x H-(Ala-Aib-Ala-Aib-Ala)₂-OMe

Boc-Ala-Aib-Ala-Aib-Ala)₂-OMe (2.046 g, 2.276 mmol) wurde mit 1.2 N HCl/AcOH (10 ml) versetzt. Nach 30 min Rühren wurde das Hydrochlorid mit Ether gefällt, abfiltriert und am Ölvakuum über KOH getrocknet.

### Ausbeute:

1.805 g (95%)

### Dünnschichtchromatographie:

R_{F}(I) = 0.50,
R_{F}(II) = 0.38,
R_{F}(III) = 0.71,
R_{F}(IV) = 0.48,
R_{F}(V) = 0.53.

### XVIII.4 Boc-Gln-(Ala-Aib-Ala-Aib-Ala)₂-OMe

Boc-Gln-OH (997 mg, 4.05 mmol) und HOBt (547 mg, 4.05 mmol) in DMF (10 ml) wurden bei -10 Grad Celsius zu HCl x H-(Ala-Aib-Ala-Aib-Ala)₂-OMe (2.250g, 2.70 mmol) und NMM (298»l, 2.70 mmol) in DMF (13 ml) gegeben. Nach Zusatz von DCC (846 mg, 4.10 mmol) in CH₂Cl₂ (2 ml) bei - 10 Grad Celsius wurde unter langsamen selbständigem Erwärmen 15 h gerührt. Nach Abbruch der Reaktion mit einigen Tropfen Eisessig wurde vom ausgefallenen DCH abzentrifugiert, der Rückstand zweimal mit wenig kaltem DMF gewaschen und das Lösungsmittel im Ölvakuum entfernt. Der Rückstand wurde in 10 ml CHCl₃/MeOH 1:1 aufgenommen und an Sephadex LH 20 in CHCl₃/MeOH (1:1) chromatographiert.

### Ausbeute:

2.60 g (94%)

### Schmelzpunkt

223 Grad Celsius (Zersetzung)

### Dünnschichtchromatographie:

R_{F}(I) = 0.66,
R_{F}(II) = 0.73,
R_{F}(III) = 0.79,
R_{F}(IV) = 0.94,
R_{F}(V) = 0.80.

### Molekulargewichtsbestimmung:

C₄₅H₇₈N₁₂O₁₅ (1027.2)

### Elementaranalyse:

| | | | |
|---|---|---|---|
| ber. | C 52.62 | H 7.65 | N 16.36 |
| gef. | C 52.65 | H 7.68 | N 16.32 |

### XVIII.5. HCl x H-Gln-(Ala-Aib-Ala-Aib-Ala)₂-OMe

Boc-Gln-(Ala-Aib-Ala-Aib-Ala)₂-OMe (2.60 g, 2.701 mmol) wurde mit 1.2 N HCl/AcOH (15 ml) versetzt. Nach 40 min wurde das Hydrochlorid unter Rühren mit Ether gefällt, abfiltriert und am Ölvakuum über KOH getrocknet.

### Ausbeute:

2.209 g (85%);

### Dünnschichtchromatographie:

R_{F}(I) = 0.48,
R_{F}(II) = 0.25,
R_{F}(III) = 0.54,
R_{F}(IV) = 0.24,
R_{F}(V) = 0.35.

### XVIII.6.Boc-Ala-Leu-Ile-Leu-Ala-Gln-(Ala-Aib-Ala-Aib-Ala)₂-OMe

Boc-Ala-Leu-Ile-Leu-Leu-Ala-OH (760 mg, 1.07 mmol) und HOBt (145 mg, 1.07 mmol) in DMF (12 ml) wurden bei Raumtemperatur zu HCl x H-Gln-(Ala-Aib-Ala-Aib-Ala)₂-OMe (818 mg, 0.85 mmol) und NMM (94»l, 0.85 mmol) in DMF (10 ml) gegeben. Nach Zugabe von DCC (227 mg, 1.10 mmol) in CH₂Cl₂ (1.5 ml) wurde 64 Stunden gerührt. Nach Abbruch der Reaktion mit wenigen Tropfen Eisessig wurde vom ausgefallenen DCH abzentrifugiert, der Rückstand zweimal mit wenig kaltem, DMF gewaschen und das Lösungsmittel im Ölvakuum entfernt. Der Rückstand wurde in 8 ml CHCl₃/MeOH (1:1) aufgenommen und an Sephadex LH 20 in CHCl₃/MeOH (1:1) chromatographiert.

### Ausbeute:

774 mg (57%);

### Schmelzpunkt:

260 Grad Celsius (Zersetzung);

### Dünnschichtchromatographie:

R_{F}(I) = 0.80,
R_{F}(II) = 0.86,
R_{F}(III) = 0.91,
R_{F}(IV) = 0.77
R_{F}(V) = 0.78.

### Aminosäurenanalyse:

Glx 1.00 (1), Ile 0.89 (1), Leu 3.10 (3), Aib 4.08 (4), Ala 7.95 (8).

### Molekulargewichtsbestimmung

C₇₅H₁₃₂N₁₈O₂₁ (1622.0)

### Elementaranalyse

| | | | |
|---|---|---|---|
| ber. | C 55.54 | H 8.20 | N 15.54 |
| gef. | C 55.58 | H 8.31 | N 15.52 |

### XIX. Herstellung von Boc-Asn-Arg(NO₂)-Arg(NO₂)-Ala-Leu-Ile-Leu-Ala-Gln-(Ala-Aib-Ala-Aib-Ala)₂-OMe

### XIX.1. HCl x H-Ala-Leu-Ile-Leu-Leu-Ala-Gln-(Ala-Aib-Ala-Aib-Ala)₂)-OMe

Boc-Ala-Leu-Ile-Leu-Leu-Ala-Gln-(Ala-Aib-Ala-Aib-Ala)₂-OMe (754 mg, 0.465 mmol) wurde mit 1.2 N HCl/AcOH (10 ml) versetzt. Nach 50 min wurde am Ölvakuum etwas eingeengt und nach Zugabe von Wasser (10 ml) lyophilisiert.

### Ausbeute:

690 mg (95%);

### Dünnschichtchromatographie:

R_{F}(I) = 0.71,
R_{F}(II) = 0.52,
R_{F}(III) = 0.78,
R_{F}(IV) = 0.56,
R_{F}(V) = 0.54.

### XIX.2. Boc-Asn-Arg(NO₂)-Arg(NO₂)-Ala-Leu-Ile-Leu-Leu-Ala-Gln-(Ala-Aib-Ala-Aib-Ala)₂-OMe

Boc-Asn-Arg(NO₂)-Arg(NO₂)-OH (634 mg, 0.995 mmol) und HOBt (135 mg, 1.13 mmol) in DMF (5 ml) wurden bei -5 Grad Celsius zu HCl x H-Ala-Leu-Ile-Leu-Leu-Ala-Gln-(Ala-Aib-Ala-Aib-Ala)₂-OMe 20 mg, 0.398 mmol) und NMM (44 Mikroliter, 400 Mikromol) in DMF (7 ml) gegeben. Nach Zusatz von DCC (217 mg, 1.05 mmol) in CH₂Cl₂ (1.5 ml) bei -5 Grad Celsius wurde unter selbständigem Erwärmen 48 h gerührt. Nach Abbruch der Reaktion mit 3 Tropfen Eisessig wurde vom ausgefallenen DCH abzentrifugiert. Die Aufarbeitung und Reinigung durch Chromatographie erfolgt, wie zuvor beschrieben.

### Ausbeute:

630 mg (74%);

### Schmelzpunkt:

195 Grad Celsius (Zersetzung);

### Dünnschichtchromatographie:

R_{F}(I) = 0.70,
R_{F}(II) = 0.51,
R_{F}(III) = 0.56,
R_{F}(IV) = 0.45,
R_{F}(V) = 0.68.

### Aminosäurenanalyse:

Asx 0.94 (1), Glx 1.00 (1), Ile 0.89 (1), Leu 3.16 (3), Arg 1.95 (2).

### Molekulargewichtsbestimmung:

C₉₁H₁₆₀N₃₀O₂₉ (2138.5)

### Elementaranalyse:

| | | | |
|---|---|---|---|
| ber. | C 51.11 | H 7.54 | N 19.65 |
| gef. | C 51.14 | H 7.60 | N 19.66 |

### XX. Herstellung des freien Eicosapeptids

### XX.1. Boc-Asn-Arg-Arg-Ala-Leu-Ile-Leu-Leu-Ala-Gln-(Ala-Aib-Ala-Aib-Ala)₂-OMe x 2HCl

Boc-Asn-Arg(NO₂)-Arg(NO₂)-Ala-Leu-Ile-Leu-Leu-Ala-Gln-(Ala-Aib-Ala-Aib-Ala)₂-OMe (350 mg, 0.164 mmol) wurde in 3 ml wasserfreiem Methanol mit 35 mg Pd/Aktivkohle sowie 12»l (0.075 mmol) 6 N HCl versetzt. Man leitete unter Rühren bei Raumtemperatur Wasserstoff durch die Lösung. Nach 20 min wurden 8 »l (49 Mikromol) und nach 35 min 7 l (42 Mikromol) 6 N HCl zugegeben. Nach ca. 50 min. Hydrierdauer war die Abspaltung nach DC-Kontrolle quantitativ. Der Katalysator wurde abfiltriert und mehrmals mit wenig Methanol gewaschen. Das Lösungsmittel wurde am Rotationsverdampfer rasch abdestilliert (Ölpumpenvakuum, Badtemperatur 25 Grad Celsius), der Rückstand in wenig Wasser aufgenommen und lyophilisiert.

### Ausbeute:

332 mg (95%);

### Dünnschichtchromatographie:

R_{F}(I) = 0.16,
R_{F}(II) = 0.11,
R_{F}(III) = 0.21,
R_{F}(IV) = 0.10.

### XX.2. H-Asn-Arg-Arg-Ala-Leu-Ile-Leu-Leu-Ala-Gln-(Ala-Aib-Ala-Aib-Ala)₂-OMe x 3 HCl

Boc-Asn-Arg-Arg-Ala-Leu-Ile-Leu-Leu-Ala-Gln-(Ala-Aib-Ala-Aib-Ala)₂-OMe x 2 HCl (600 mg, 0.283 mmol) wurde mit 1.2 N HCl/AcOH (5 ml) versetzt. Nach 30 min wurde etwas am Rotationsverdampfer eingeengt, mit Wasser (10 ml) versetzt und lyophilisiert.

### Ausbeute:

564 mg (97%);

### Schmelzpunkt:

245 Grad Celsius (Zersetzung)

### Dünnschichtchromatographie:

R_{F}(I) = 0.11

### Molekulargewichtsbestimmung:

C₈₆H₁₅₇N₂₈O₂₃Cl₃ (2057.7)

### Elementaranalyse:

| | | | | |
|---|---|---|---|---|
| ber. | C 50.20 | H 7.69 | N 19.06 | Cl 5.17 |
| gef. | C 50.31 | H 7.78 | N 18.95 | Cl 5.28 |

### Materialien und Methoden der Experimente

### Chemikalien

Lösungsmittel p.a. wurden von der Fa. Merck bezogen, ansonsten nach üblichen Methoden getrocknet und destilliert. N-Methyl-morpholin (Merck) wurde zur Entfernung sek. Amine über Ninhydrin destilliert. 1-Hydroxybenzotriazol und Dicyclohexylcarbodiimid stammten ebenfalls von Merck. Alle L-Aminosäurederivate wurden von der Fa. Bachem bezogen. Boc-Aib-OH und H-Aib-OMe x HCl wurden nach Literaturvorschriften synthetisiert.

### Dünnschichtchromatographie

Es wurden Fertigplatten Kieselgel 60 F₂₅₄ (Fa. Merck) und folgende Fließmittel verwendet:
(I) 1-Butanol/Eisessig/Wasser 3:1:1
(II) Chloroform/Methanol/Eisessig/Wasser 65:25:3:4
(III) Chloroform/Methanol/konz. Ammoniak/Wasser 65:35:3:4
(IV) Chloroform/Methanol/Wasser 65:25:4
(V) Chloroform/Methanol 1:1
Als Sprühreagentien wurden eingesetzt: Ninhydrinreagens, Chlor/4,4'-Bis(dimethylamino)diphenylmethan (TDM-Reagens) und Sakaguchi-Reagens. Als Referenz diente Dicylohexylharnstoff mit folgenden Werten:
R_{F}(I) 0.91, R_{F}(II) 0.82, R_{F}(III) 0.92, R_{F}(IV) 0.81, R_{F}(V) 0.83.

### Aminosäurenanalysen

Zur Feststellung der Identität der Zwischenstufen wurden von den geschützten Peptiden jeweils ca. 200 Mikrogramm-Proben in 6N HCl 24 Stunden bei 110 Grad Celsius hydrolysiert. Zwischenstufen und Zielsequenz des Hexapeptid-Segments, das die Leu-Leu-Bindung enthält, wurden unter sonst gleichen Bedingungen 72 Stunden hydrolysierv. Die Aminosäurenanalysen wurden mit einem Biotronik-Aminosäurenanalysator LC 6000 E unter Verwendung der Standard-Programmierung durchgeführt.

### Racematbestimmung

Die hydrolysierten Aminosäuren wurden als Pentafluorpropionylaminosäure-n-propylester derivatisiert, und die Enantiomeren an Glaskapillarsäulen mit Chirasil-Val gaschromatographisch getrennt. Die angegebenen Prozentanteile an D-Aminosäuren sind nicht auf hydrolysebedingte Racemisierung korrigiert.

### Elementaranalysen

Es wurden C,H,N-Einfachbestimmungen an einem Elemental-Analyzer Modell 1104 (Carlo Erba, Mailand) durchgeführt.

### Schmelzpunkte

Schmelzpunkte wurden nach Tottoli ermittelt und sind nicht korrigiert.

### Aufnahme der Spektren

¹³C-NMR-Spektren: 30 mg des geschützten Eicosapeptids wurden in 400 Mikrolitern ¹²C²HCl₃/¹²C²H₃O²H (1:1) (Fa. Merck) gelöst und im NMR-Spektrometer WM 400 (Fa. Bruker) 12 h bei 30 °C vermessen. Circulardichroismus-Spektren: Lösungen des freien Eicosapeptids (c = 1-1.7 mg/ml) in Ethanol, Trifluorethanol, Methanol, 1.1.1.3.3.3-Hexafluorpropanol-(2), Wasser sowie Ethanol/Wasser-Mischungen wurden mit dem Dichrograph II (Fa. Jouhan-Roussel) vermessen.

### Chromatographische Reinigung

Die geschützten Peptid-Zwischenstufen wurden nach Beendigung der Kupplungsreaktion und Abziehen des Lösungsmittels am Ölvakuum durch Zugabe des gleichen Volumens an CHCl₃/MeOH 1:1 gelöst, vom Dicyclohexylharnstoff abzentrifugiert und an Sephadex LH 20 chromatographiert: Säule 3 x 115 cm; Elutionsmittel CHCl₃/MeOH 1:1; Auftragsmenge 35 ml; Fließgeschwindigkeit 8,40 ml/10 min. Die 3 ml-Fraktionen wurden dünnschichtchromatographisch im System II untersucht (TDM-Reagens). Die Peptide erschienen im Elutionsvolumen 165 -190 ml. Die Fraktionen wurden vereinigt, das Lösungsmittel im Vakuum abgezogen, und der Rückstand über P₂O₅ getrocknet. Die Aminosäurenanalyse lieferte die erwarteten Werte und einen Peptidgehalt von 92-96%.

### Immunisierungsversuche

Wir haben erstmals ein B-Zell-Mitogen, das zugleich ein hervorragender Carrier und ein stark wirksames Adjuvans ist, kovalent mit synthetischen antigenen Determinanten verknüpft. Dazu verwendeten wir u.a. das synthetische Lipopeptid S-(2,3-Bis(palmitoyloxy)propyl)-N-palmitoylcysteinyl-serin (Pam₃Cys-Ser), das den N-Terminus des Lipoproteins aus der äußeren Membran von Escherichia coli darstellt. Die in kovalenter Verbindung mit einem Antigen besonders ausgeprägt amphiphilen Eigenschaften gewährleisten einerseits eine stabile Verankerung der drei Fettsäurereste tragenden S-Glyceryl-Verbindung in der Lipidschicht der Zellmembran. Andererseits wird dadurch das meist polarere Antigen (bzw. Hapten) in der äußeren hydrophilen Schicht der Membran präsentiert. Da die aktivierende Wirkung des Lipoproteins allein von dessen N-terminalem Teil bestimmt wird, bleibt in allen Konjugaten, die Pam₃Cys-Ser oder Analoga tragen, dessen immunstimulierende Wirkung erhalten.

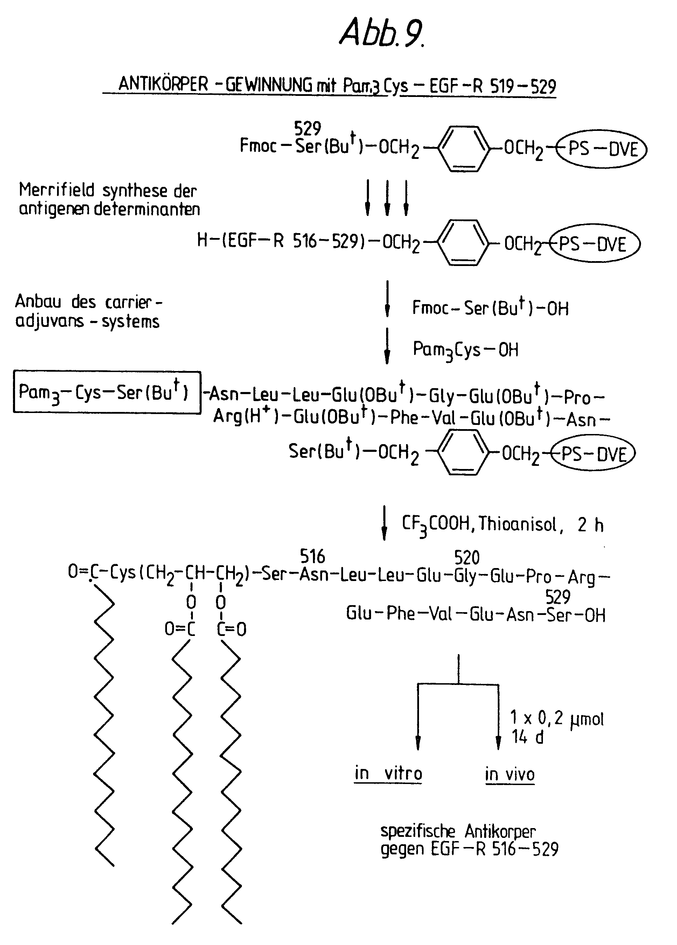

Als Beispiel führen wir die Anwendung des Konzepts zur Erzeugung von spezifischen Antikörpern gegen den Epidermal Growth Factor-Rezeptor (EGF-R) Fig. 9 an. Dazu wurde durch computerunterstützte Epitopsuche die extracytoplasmatische Region 516-529 ausgewählt, durch Merrifield-Synthese aufgebaut und zuletzt Fmoc-Ser(Bu^{t})-OH und dann Pam₃Cys-OH angebaut. Nach Abspaltung vom Harz wurde das analytisch als einheitlich befundene Konjugat ohne weitere Zusätze in einer einmaligen Dosis Mäusen i.p. appliziert. Mittels ELISA Assays wurden bereits nach 2 Wochen hohe Titer an spezifischen Antikörpern gegen das Tetradekapeptid festgestellt. Wesentlich ist, daß bei Kontrollversuchen durch das allein offensichtlich schwach immunogene Tetradekapeptid keine Antikörpertiter erhalten wurden.

Da Pam₃Cys-Konjugate ebenfalls in Zellkulturen stark immunogen sind, können durch in vitro Immunisierung auf eine rasche und elegante Weise konventionelle und monoklonale Antikörper auch gegen schwach immunogene Verbindungen erhalten werden.

Die Vorteile unseres Konzepts in Verbindung mit Zellkulturen sind: einfache Herstellung eines chemisch eindeutig definierten Antigen-Adjuvans-Konjugats in beliebiger Menge, im Gegensatz zu anderen Konjugaten einmalige Applikation ohne mehrfaches "Boostern", hohe Effizienz in vivo und in vitro. Die erhebliche Einsparung von Versuchstieren, häufig sogar ein völliger Verzicht auf in vivo Immunisierungen und eine drastische Zeitersparnis insbesondere bei gentechnologischem Arbeiten liegen auf der Hand. Die Versuche können auch mit Humanzellkultursystemen durchgeführt werden.

### Beispiel für eine in vivo Immunisierung:

6 bis 10 Wochen alte Balb/c-Mäuse wurden durch einmalige i.p. Applikation von 50 Mikrogramm und 500 Mikrogramm (0,2 ml einer 10⁻¹ bis 10⁻² molaren Lösung von kovalent an Antigen gekoppeltem Adjuvans) Pam₃Cys-Ser-(EGF-R 515-529) immunisiert. Als Kontrollen dienten Antigen, Adjuvans und eine Mischung von Antigen und Adjuvans jeweils in vergleichbaren molaren Mengen, sowie Medium. Zwei Wochen nach der Injektion wurde den Mäusen aus dem retroorbitalen Venenplexus Blut zur Gewinnung von Serum entnommen und der Antikörpertiter durch ELISA bestimmt.

Analoge Immunisierungen können auch durch andere Applikationen z.B. i.v., oral, rektal, i.m., s.c. erreicht werden.

Die Bildung spezifischer Antikörper ohne Freund's Adjuvans gegen das Tetradekapeptid EGF-R 516-529 nach in vivo Immunisierung wurde untersucht.

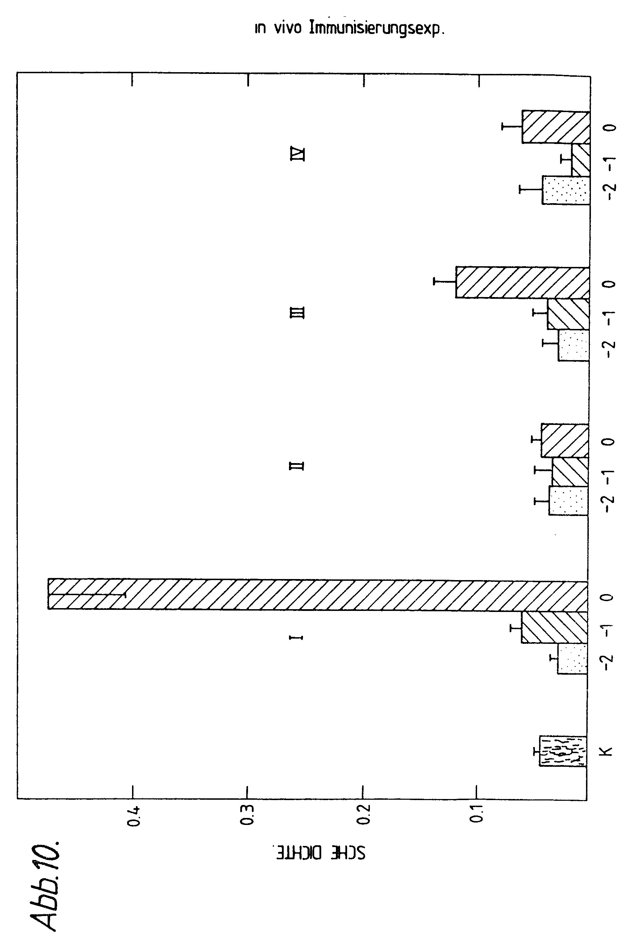

Balb/c-Mäuse wurden, wie in Fig. 10 gezeigt, mit
I. Konjugat Pam₃Cys-Ser (EGF-R 516-526)
II. Freies Tetradekapeptid EGF-R 516-529, alleine
III.Pam₃Cys-Ser alleine
IV. freies Tetradekapeptid (EGF-R 516-529) gemeinsam in Mischung mit Pam₃Cys-Ser
einmalig i.p. mit 0,2 Mikromol des Konjugats immunisiert. Der Antikörpertiter wurde mittels ELISA Assay bestimmt. (Ordinate OD bei 405 nm) (Fig.10).

14 Tage nach der Immunisierung wurden die Mäuse aus der Augenvene geblutet und das gewonnene Serum in ELISA eingesetzt. Die Werte ergeben sich aus dem Mittelwert (3-5 Mäuse) der Differenz zwischen den ELISA-Werten von PEP 14 - BSA Konjugat und BSA. (Fig. 10)

Es zeigt sich, daß nur bei Verwendung des erfindungsgemäßen Menmbrananker-Wirkstoffkonjugats drastisch erhöhte, bisherige Verfahren um ein Vielfaches an Wirdsamkeit übertreffende Antikörperkonzentrationen gefunden werden.

### Beispiel für eine in vitro Immunisierung

Milzzellen aus Mäusen wurden jeweils in Gegenwart von Konjugat Pam₃Cys-Ser-(EGF-R 515-529), von Adjuvans Pam₃Cys-Ser, von Tetradekapeptid EGF-R 516-529, einer Mischung von Antigen und Adjuvans und Medium 5 Tage lang kultiviert.

Die Lymphozyten wurden bei einer Zelldichte von 2,5 x 10⁶/ml 48 h lang in 0,2 ml Aliquote, in RPMI-1640 Medium, angereichert mit 10% hitzeinaktiviertem FCS, Glutamin (2mM), Penicillin (100 U/ml), Streptomycin (100»g/ml) und 2-Mercaptoethanol (5 x 10⁻⁵ M) kultiviert.

Die Überstande wurden zur Untersuchung auf spezifische Antikörper durch ELISA-Assay gewonnen.

### Mitogene Aktivierung von Maus-Milzzellen

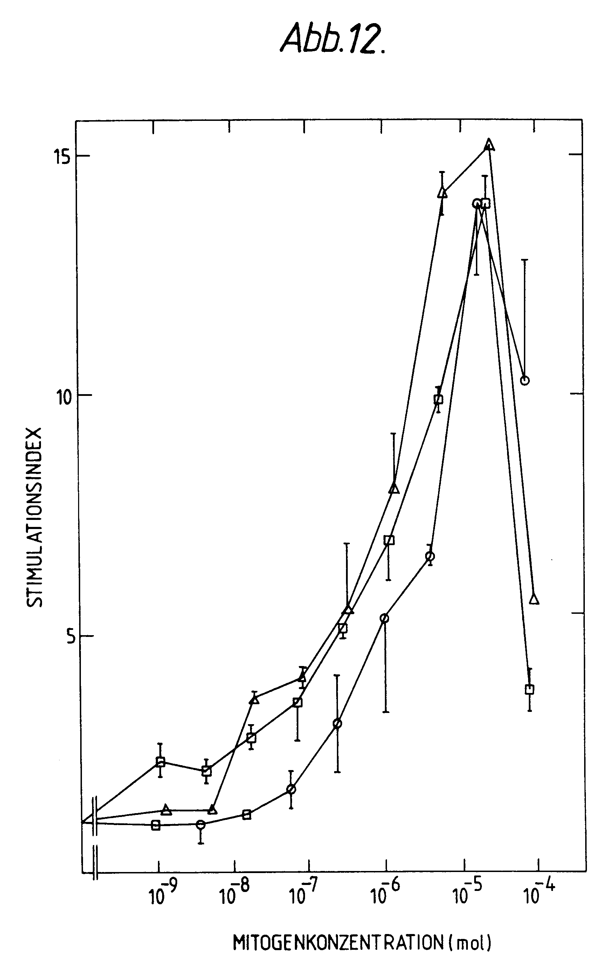

Die mitogene Aktivierung von Balb/c-Milzzellen durch Pam₃Cys-Ser-(Lys)₄-FITC (Kreise), Pam₃Cys-Ser-(Lys)₄-OH x 3 HCl (Dreiecke) und Pam₃Cys-Ser-(Lys)₄-OH x 2 TFE (Vierecke) ist aus Fig. 12 ersichtlich. Die Zellkultivierungsbedingungen sind beschrieben. (Z.Immunforsch. 153, 1977, S.11 ff. und Eur. J. Biochem. 115 ,1981). In der Zeichnung ist auf der Ordinate der Stimulationsindex des Einbaus von ³H-Thymidin in die DNA (cpm-Werte der Inkorporation/cpm-Werte der Kontrolle ohne Mitogen) gegen die Konzentration des eingesetzten Wirkstoffes aufgetragen.

### Vergleich in vivo/in vitro

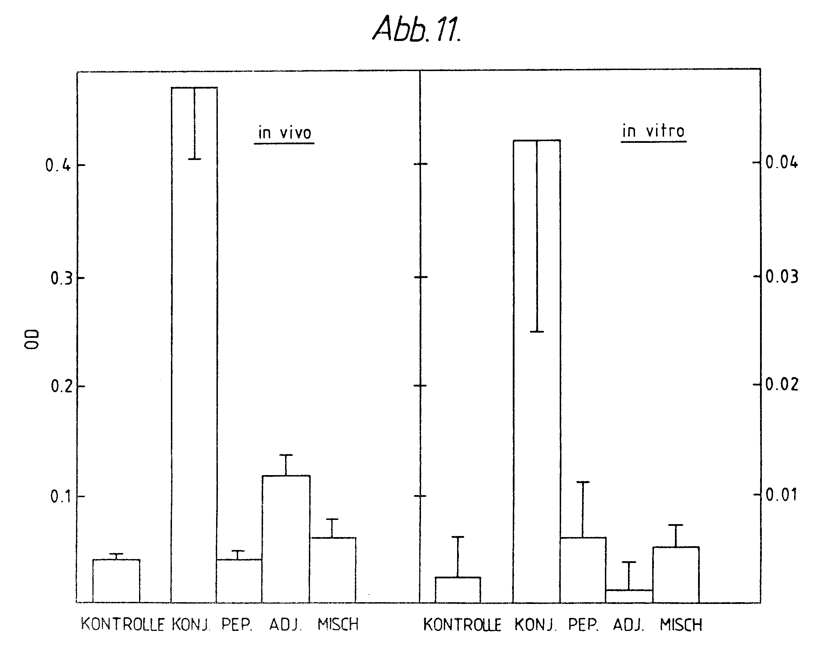

In Fig. 11 ist der oben aufgeführte in vivo Versuch einem in vitro Experiment gegenübergestellt:
In vitro-Versuch in Mikrotiterplatten: Zelldichte: 2,5 x 10⁶ Zellen/ml; Substanzkonzentration: 5 x 10⁻⁷ mmolar; Inkubationsbedingungen: 37 °C, 5 % CO₂, 5 Tage.
- Conj.: : Konjugat Pam₃Cys-Ser-(EGF-R 515-529)
- Pep: : Tetradekapeptid EGF-R 516-529
- Adj.: : Pam₃Cys-Ser
- Mix: : Mischung von freiem Tetradekapeptid EGF-R 516-529 und Pam₃Cys-Ser
Auch in vitro ergibt sich der drastische Anstieg der Antikörperkonzentration, wodurch die Einsetzbarkeit von Zellkulturen, insbesondere für die Herstellung von Antikörpern, erheblich erweitert wird.

## Patentansprüche (Patentansprüche für folgende(n) Vertragsstaat(en): BE, CH, DE, FR, GB, IT, LI, LU, NL, SE)

1. Membrananker-Wirkstoffkonjugat, dadurch gekennzeichnet, daß es aus mindestens einer Membranankerverbindung und mindestens einem kovalent an die Membranankerverbindung(en) gebundenen Wirkstoff besteht, wobei die Membranankerverbindung eine Verbindung der allgemeinen Formel I ist: 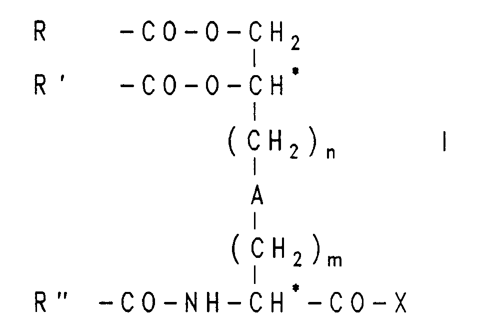 wobei A = Schwefel, Sauerstoff, Disulfid (-S-S-), Methylen (-CH₂-) oder -NH-sein kann; n = 0 bis 5; m = 1 oder 2; C* = asymmetrisches Kohlenstoffatom mit R oder S Konfiguration R, R', R'' gleich oder unterschiedlich sind und eine Alkyl-, Alkenyl- oder Alkinyl-Gruppe mit 7 bis 25 Kohlenstoffatomen oder Wasserstoff ist, welche gegebenenfalls mit Hydroxi-, Amino-, Oxo-, Acyl-, Alkyl- oder Cycloalkyl-Gruppen substituiert sein kann und X ein Wirkstoff oder eine Spacer-Wirkstoffgruppe ist
mit Ausnahme der Verbindungen der Formel I' 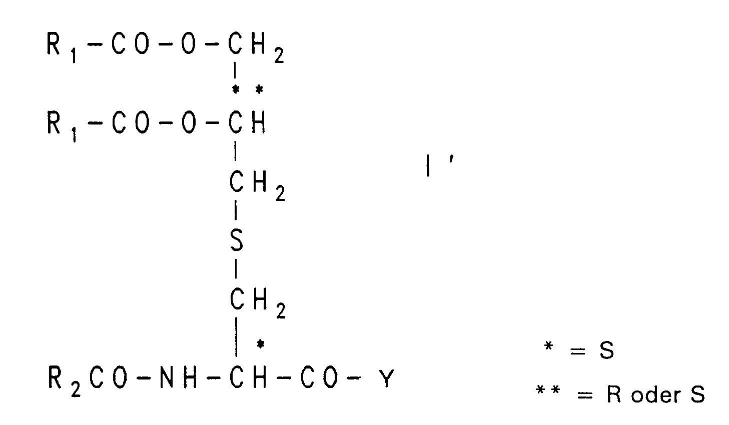 worin R₁ und R₂ je einen gesättigten oder ungesättigten, aliphatischen oder gemischt aliphatisch-cycloaliphatischen, gegebenenfalls auch durch Sauerstoffunktionen substituierten, Kohlenwasserstoffrest mit 11 bis 21 C-Atomen und Y eine peptidisch gebundene natürliche aliphatische Aminosäure mit freier, veresterter oder amidierter Carboxylgruppe, oder eine Aminosäurensequenz von 2 bis 10 natürlichen aliphatischen Aminosäuren, deren terminale Carboxylgruppe in freier, veresterter oder amidierter Form vorliegt, bedeuten, wobei die mit * bzw. ** bezeichneten Asymmetriezentren die absolute S- oder S- oder R-Konfiguration besitzen, und Gemische der an den ** C-Atomen epimeren R- und S-Verbindungen.

2. Membrananker-Wirkstoffkonjugat gemäß Anspruch 1, dadurch gekennzeichnet, daß die Membranankerverbindung Pam₃Cys oder Pam₃Cys-Ser oder ein Pam₃Cys-Peptid mit 1 bis 10 Aminosäuren ist.

3. Membrananker-Wirkstoffkonjugat gemäß Anspruch 1 oder 2, dadurch gekennzeichnet, daß der Wirkstoff ein Antigen, wie beispielsweise eine niedermolekulare Partialsequenz eines Proteins oder Proteids, beispielsweise eines Glycoproteins, eines Virushüllproteins, Bakterienzellwandproteins oder Proteins von Protozoen (antigene Determinate, Epitop), Bakterienmembranbestandteile, wie Muramyldipeptid, Lipopolysaccharid, ein Antibiotikum, ein Hormon, ein Nukleosid, ein Nukleotid, eine Nukleinsäure, ein Enzym, ein Enzymsubstrat, ein Enzyminhibitor, Biotin, Avidin, Polyethylenglykol, Peptidwirkstoffe wie Tuftsin; Polylysin, ein Alkaloid, Steroid, biogenes Amin, Vitamin, ein Toxin wie beispielsweise Digoxin, Phalloidin, Amanitin oder Tetrodoxin.

4. Membrananker-Wirkstoffkonjugat gemäß einem der vorangehenden Ansprüche, dadurch gekennzeichnet, daß die Membranankerverbindung und der Wirkstoff über einen Crosslinker miteinander kovalent verbunden sind, beispielsweise über ein Polyethylenglykol.

5. Verfahren zum Herstellen von Membranankerverbindungen gemäß einem oder mehreren der Ansprüche 1 bis 4, dadurch gekennzeichnet, daß das mit Schutzgruppen in an sich bekannter Weise an den Funktionen, an denen keinerlei Reaktion stattfinden soll, geschützte Peptid mittels bekannter Kupplungsverfahren an einen festen oder löslichen Träger, wie einem Polymer (z. B. Merrifield-Harz), synthetisiert wird, daß die derart synthetisierten, am Träger gebundenen Peptide, über N-Termini oder Seitenfunktionen des Peptids mit dem Membranankerverbindung kovalent gebunden werden; daß das derart hergestellte Peptidkonjugat isoliert wird, dadurch, daß die Schutzgruppen sowie die Bindung Peptid/Träger in an sich bekannter Weise gespalten werden und somit das Membranankerpeptid oder das Membranankerwirkstoffkonjugat gewonnen wird.

6. Verfahren gemäß Anspruch 5, dadurch gekennzeichnet, daß die Verknüpfung Peptid/Membrananker durch eine Kondensation, Addition, Substitution, Oxidation, z. B. Disulfidbildung, hergestellt wird.

7. Verwendung der Verbindungen gemäß einem oder mehreren der Ansprüche 1 bis 4 zur Herstellung konventioneller und monoklonaler Antikörper in vivo und in vitro.

8. Verwendung der Verbindungen gemäß einem oder mehreren der Ansprüche 1 bis 4 zur Herstellung synthetischer Vakzine.

9. Verwendung der Verbindungen gemäß einem oder mehreren der Ansprüche 1 bis 4 für Liposomenpräparate.

10. Verwendung der Verbindungen gemäß einem oder mehreren der Ansprüche 1 bis 4 als Zusatz zu Lebensmittels oder Futterstoffen im Human- und Tierbereich, sowie als Zusatz zu Kulturmedien für Mikroorganismen und allgemein für Zellkulturen.

11. Verwendung eines Membrananker-Wirkstoffkonjugats, dadurch gekennzeichnet, daß es aus mindestens einer Membranankerverbindung und mindestens einem kovalent an die Membranankerverbindung(en) gebundenen Wirkstoffen besteht, wobei die Membranankerverbindung eine Verbindung der allgemeinen Formel I ist: 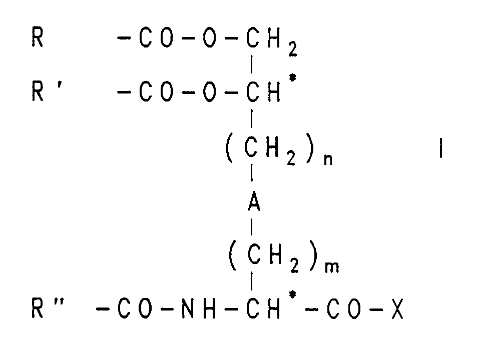 wobei A = Schwefel, Sauerstoff, Disulfid (-S-S-), Methylen (-CH₂-) oder -NH- sein kann; n = 0 bis 5; m = 1 oder 2; C* = asymmetrisches Kohlenstoffatom mit R oder S Konfiguration R, R', R'' gleich oder unterschiedlich sind und eine Alkyl-, Alkenyl- oder Alkinyl-Gruppe mit 7 bis 25 Kohlenstoffatomen oder Wasserstoff ist, welche gegebenenfalls mit Hydroxi-, Amino-, Oxo-, Acyl-, Alkyl- oder Cycloalkyl-Gruppen substituiert sein kann zur Herstellung eines Arzneimittels zur Erzeugung einer Immunantwort gegen den Wirkstoff.

## Patentansprüche (Patentansprüche für folgende(n) Vertragsstaat(en): AT)

1. Verfahren zur Herstellung eines Membrananker-Wirkstoffkonjugats, bestehend aus mindestens einer Membranankerverbindung und mindestens einem kovalent an die Membranankerverbindung(en) gebundenen Wirkstoff, wobei die Membranankerverbindung eine Verbindung der allgemeinen Formel I ist: 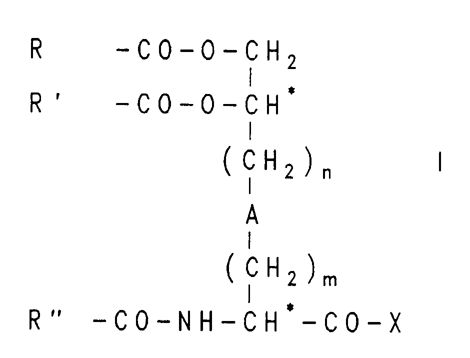 wobei A = Schwefel, Sauerstoff, Disulfid (-S-S-), Methylen (-CH₂-) oder -NH- sein kann; n = 0 bis 5; m = 1 oder 2; C* = asymmetrisches Kohlenstoffatom mit R oder S Konfiguration R, R', R'' gleich oder unterschiedlich sind und eine Alkyl-, Alkenyl- oder Alkinyl-Gruppe mit 7 bis 25 Kohlenstoffatomen oder Wasserstoff ist, welche gegebenenfalls mit Hydroxi-, Amino-, Oxo-, Acyl-, Alkyl- oder Cycloalkyl-Gruppen substituiert sein kann und X ein Wirkstoff oder eine Spacer-Wirkstoffgruppe ist
mit Ausnahme der Verbindungen der Formel I' 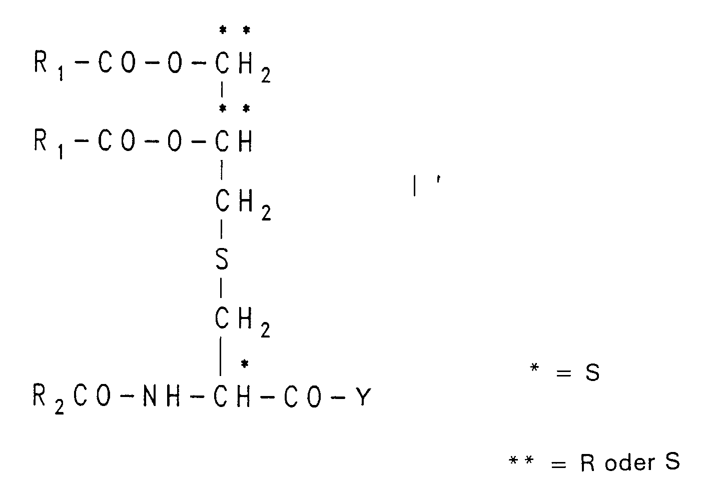 worin R₁ und R₂ je einen gesättigten oder ungesättigten, aliphatischen oder gemischt aliphatisch-cycloaliphatischen, gegebenenfalls auch durch Sauerstoffunktionen substituierten, Kohlenwasserstoffrest mit 11 bis 21 C-Atomen und Y eine peptidisch gebundene natürliche aliphatische Aminosäure mit freier, veresterter oder amidierter Carboxylgruppe, oder eine Aminosäurensequenz von 2 bis 10 natürlichen aliphatischen Aminosäuren, deren terminale Carboxylgruppe in freier, veresterter oder amidierter Form vorliegt, bedeuten, wobei die mit * bzw. ** bezeichneten Asymmetriezentren die absolute S- oder S- oder R-Konfiguration besitzen, und Gemische der an den ** C-Atomen epimeren R- und S-Verbindungen, dadurch gekennzeichnet, daß das mit Schutzgruppen in an sich bekannter Weise an den Funktionen, an denen keinerlei Reaktion stattfinden soll, geschützte Peptid mittels bekannter Kupplungsverfahren an einen festen oder löslichen Träger, wie einem Polymer (z. B. Merrifield-Harz), synthetisiert wird, daß die derart synthetisierten, am Träger gebundenen Peptide, über N-Termini oder Seitenfunktionen des Peptids mit dem Membranankerverbindung kovalent gebunden werden; daß das derart hergestellte Peptidkonjugat isoliert wird, dadurch, daß die Schutzgruppen sowie die Bindung Peptid/Träger in an sich bekannter Weise gespalten werden und somit das Membranankerpeptid oder das Membranankerwirkstoffkonjugat gewonnen wird.

2. Verfahren gemäß Anspruch 1, dadurch gekennzeichnet, daß die Membranankerverbindung Pam₃Cys oder Pam₃Cys-Ser oder ein Pam₃Cys-Peptid mit 1 bis 10 Aminosäuren ist.

3. Verfahren gemäß Anspruch 1 oder 2, dadurch gekennzeichnet, daß der Wirkstoff ein Antigen, wie beispielsweise eine niedermolekulare Partialsequenz eines Proteins oder Proteids, beispielsweise eines Glycoproteins, eines Virushüllproteins, Bakterienzellwandproteins oder Proteins von Protozoen (antigene Determinate, Epitop), Bakterienmembranbestandteile, wie Muramyldipeptid, Lipopolysaccharid, ein Antibiotikum, ein Hormon, ein Nukleosid, ein Nukleotid, eine Nukleinsäure, ein Enzym, ein Enzymsubstrat, ein Enzyminhibitor, Biotin, Avidin, Polyethylenglykol, Peptidwirkstoffe wie Tuftsin; Polylysin, ein Alkaloid, Steroid, biogenes Amin, Vitamin, ein Toxin wie beispielsweise Digoxin, Phalloidin, Amanitin oder Tetrodoxin.

4. Verfahren gemäß einem der vorangehenden Ansprüche, dadurch gekennzeichnet, daß die Membranankerverbindung und der Wirkstoff über einen Crosslinker miteinander kovalent verbunden sind, beispielsweise über ein Polyethylenglykol.

5. Verfahren gemäß einem oder mehreren der Ansprüche 1-4, dadurch gekennzeichnet, daß die Verknüpfung Peptid/Membrananker durch eine Kondensation, Addition, Substitution, Oxidation, z. B. Disulfidbildung, hergestellt wird.

6. Verwendung der Verbindungen gemäß einem oder mehreren der Ansprüche 1 bis 4 zur Herstellung konventioneller und monoklonaler Antikörper in vivo und in vitro.

7. Verwendung der Verbindungen gemäß einem oder mehreren der Ansprüche 1 bis 4 zur Herstellung synthetischer Vakzine.

8. Verwendung der Verbindungen gemäß einem oder mehreren der Ansprüche 1 bis 4 für Liposomenpräparate.

9. Verwendung der Verbindungen gemäß einem oder mehreren der Ansprüche 1 bis 4 als Zusatz zu Lebensmittels oder Futterstoffen im Human- und Tierbereich, sowie als Zusatz zu Kulturmedien für Mikroorganismen und allgemein für Zellkulturen.

10. Verwendung eines Membrananker-Wirkstoffkonjugats, dadurch gekennzeichnet, daß es aus mindestens einer Membranankerverbindung und mindestens einem kovalent an die Membranankerverbindung(en) gebundenen Wirkstoffen besteht, wobei die Membranankerverbindung eine Verbindung der allgemeinen Formel I ist: 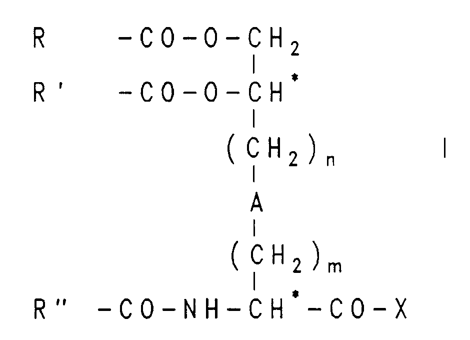 wobei A = Schwefel, Sauerstoff, Disulfid (-S-S-), Methylen (-CH₂-) oder -NH- sein kann; n = 0 bis 5; m = 1 oder 2; C* = asymmetrisches Kohlenstoffatom mit R oder S Konfiguration R, R', R'' gleich oder unterschiedlich sind und eine Alkyl-, Alkenyl- oder Alkinyl-Gruppe mit 7 bis 25 Kohlenstoffatomen oder Wasserstoff ist, welche gegebenenfalls mit Hydroxi-, Amino-, Oxo-, Acyl-, Alkyl- oder Cycloalkyl-Gruppen substituiert sein kann zur Herstellung eines Arzneimittels zur Erzeugung einer Immunantwort gegen den Wirkstoff.

## Claims (Claims for the following Contracting State(s): BE, CH, DE, FR, GB, IT, LI, LU, NL, SE)

1. A membrane anchor/active compound conjugate which comprises at least one membrane anchor compound and at least one active compound covalently bonded to the membrane anchor compound(s), where the membrane anchor compound is a compound of the formula I: 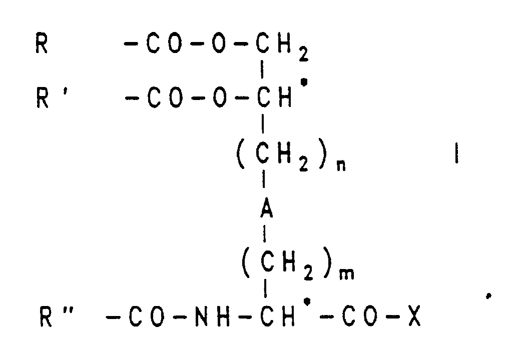 it being possible for A to be sulfur, oxygen, disulfide (-S-S-), methylene (-CH₂-) or -NH-; n being 0 to 5; m being 1 or 2; C* being an asymmetric carbon atom with R or S configuration; R, R' and R'' being identical or different and being an alkyl, alkenyl or alkynyl group having 7 to 25 carbon atoms or hydrogen, which can optionally be substituted by hydroxyl, amino, oxo, acyl, alkyl or cycloalkyl groups, and X being an active compound or a spacer-active compound group, with the exception of the compounds of the formula I' 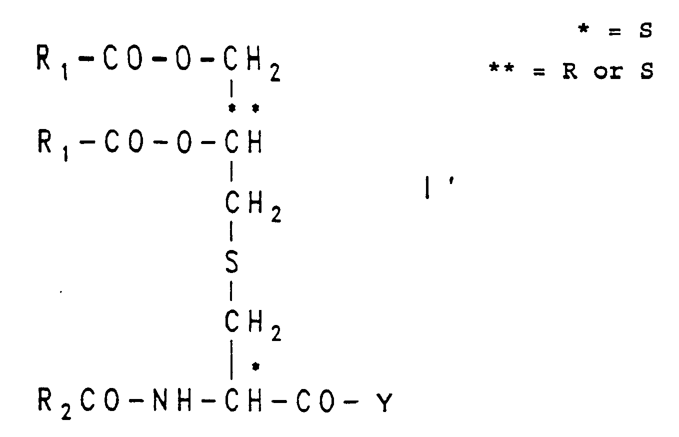 in which R₁ and R₂ are each a saturated or unsaturated, aliphatic or mixed aliphatic-cycloaliphatic hydrocarbon radical which is optionally also substituted by oxygen functional groups and has 11 to 21 carbon atoms and Y is a peptide-bonded natural aliphatic amino acid with free, esterified or amidated carboxyl group, or an amino-acid sequence of 2 to 10 natural aliphatic amino acids whose terminal carboxyl group is in free, esterified or amidated form, where the centers of asymmetry identified by * and ** have the absolute S or S or R configuration, and mixtures of the R and S compounds which are epimeric at the ** carbon atoms.

2. A membrane anchor/active compound conjugate as claimed in claim 1, wherein the membrane anchor compound is Pam₃-Cys or Pam₃Cys-Ser or a Pam₃Cys-peptide having 1 to 10 amino acids.

3. A membrane anchor/active compound conjugate as claimed in claim 1 or 2, wherein the active compound is an antigen such as, for example, a low molecular weight partial sequence of a protein or conjugated protein, for example of a glycoprotein, of a viral coat protein, of a bacterial cell wall protein or of a protein of protozoa (antigenic determinants, epitope), constituents of bacterial membranes, such as muramyldipeptide, lipopolysaccharide, an antibiotic, a hormone, a nucleoside, a nucleotide, a nucleic acid, an enzyme, an enzyme substrate, an enzyme inhibitor, biotin, avidin, polyethylene glycol, peptidic active compounds such as tuftsin; polylysine, an alkaloid, steroid, biogenic amine, vitamin or a toxin such as, for example, digoxin, phalloidin, amanitin or tetrodotoxin.

4. A membrane anchor/active compound conjugate as claimed in one of the preceding claims, wherein the membrane anchor compound and the active compound are covalently bonded together via a crosslinker, for example via a polyethylene glycol.

5. A process for the preparation of membrane anchor compounds as claimed in one or more of claims 1 to 4, which comprises synthesis of the peptide, which is protected with protective groups in a manner known per se on the functional groups at which no reaction is to take place, by means of known coupling processes on a solid or soluble carrier, such as a polymer (for example Merrifield resin), covalent bonding of the carrier-bound peptides, which have been synthesized in this way, via N-termini or sidegroups of the peptide to the membrane anchor compound; isolation of the peptide conjugate, which has been prepared in this way, by cleavage of the protective groups and the peptide/carrier bond in a manner known per se, and thus the membrane anchor/peptide or the membrane anchor/active compound conjugate being obtained.

6. The process as claimed in claim 5, wherein the peptide/membrane anchor linkage is produced by condensation, addition, substitution or oxidation, for example disulfide formation.

7. The use of the compounds as claimed in one or more of claims 1 to 4 for the preparation of conventional and monoclonal antibodies in vivo and in vitro.

8. The use of the compounds as claimed in one or more of claims 1 to 4 for the preparation of synthetic vaccines.

9. The use of the compounds as claimed in one or more of claims 1 to 4 for liposome preparations.

10. The use of the compounds as claimed in one or more of claims 1 to 4 for addition to human foodstuffs or animal feeds, and for addition to culture media for microorganisms and generally for cell cultures.

11. The use of a membrane anchor/active compound conjugate which comprises at least one membrane anchor compound and at least one active compound covalently bonded to the membrane anchor compound(s), where the membrane anchor compound is a compound of the formula I: 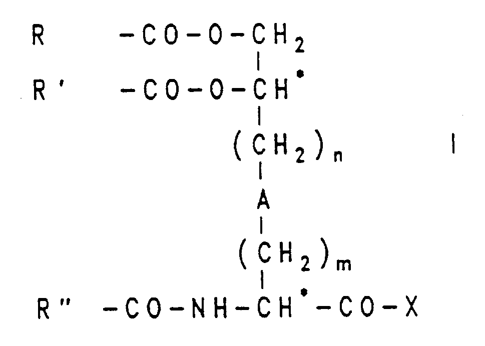 it being possible for A to be sulfur, oxygen, disulfide (-S-S-), methylene (-CH₂-) or -NH-; n being 0 to 5; m being 1 or 2; C* being an asymmetric carbon atom with R or S configuration; R, R' and R'' being identical or different and being an alkyl, alkenyl or alkynyl group having 7 to 25 carbon atoms or hydrogen, which can optionally be substituted by hydroxyl, amino, oxo, acyl, alkyl or cycloalkyl groups, for the preparation of a pharmaceutical for generating an immune response toward the active compound.

## Claims (Claims for the following Contracting State(s): AT)

1. A process for the preparation of a membrane anchor/active compound conjugate comprising at least one membrane anchor compound and at least one active compound covalently bonded to the membrane anchor compound(s), where the membrane anchor compound is a compound of the formula I: 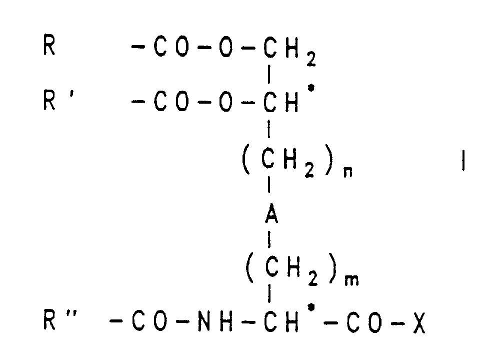 it being possible for A to be sulfur, oxygen, disulfide (-S-S-), methylene (-CH₂-) or -NH-; n being 0 to 5; m being 1 or 2; C* being an asymmetric carbon atom with R or S configuration; R, R' and R'' being identical or different and being an alkyl, alkenyl or alkynyl group having 7 to 25 carbon atoms or hydrogen, which can optionally be substituted by hydroxyl, amino, oxo, acyl, alkyl or cycloalkyl groups, and X being an active compound or a spacer-active compound group, with the exception of the compounds of the formula I' 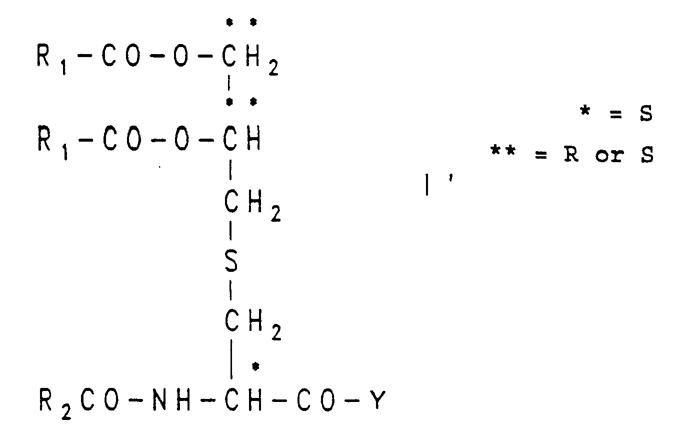 in which R₁ and R₂ are each a saturated or unsaturated, aliphatic or mixed aliphatic-cycloaliphatic hydrocarbon radical which is optionally also substituted by oxygen functional groups and has 11 to 21 carbon atoms and Y is a peptide-bonded natural aliphatic amino acid with free, esterified or amidated carboxyl group, or an amino-acid sequence of 2 to 10 natural aliphatic amino acids whose terminal carboxyl group is in free, esterified or amidated form, where the centers of asymmetry identified by * and ** have the absolute S or S or R configuration, and mixtures of the R and S compounds which are epimeric at the ** carbon atoms, which comprises synthesis of the peptide, which is protected with protective groups in a manner known per se on the functional groups at which no reaction is to take place, by means of known coupling processes on a solid or soluble carrier, such as a polymer (for example Merrifield resin); covalent bonding of the carrier-bound peptides, which have been synthesized in this way, via N-termini or sidegroups of the peptide to the membrane anchor compound; isolation of the peptide conjugate, which has been prepared in this way, by cleavage of the protective groups and the peptide/carrier bond in a manner known per se, and thus the membrane anchor peptide or the membrane anchor/active compound conjugate being obtained.

2. The process as claimed in claim 1, wherein the membrane anchor compound is Pam₃-Cys or Pam₃Cys-Ser or a Pam₃Cys-peptide having 1 to 10 amino acids.

3. The process as claimed in claim 1 or 2, wherein the active compound is an antigen such as, for example, a low molecular weight partial sequence of a protein or conjugated protein, for example of a glycoprotein, of a viral coat protein, of a bacterial cell wall protein or of a protein of protozoa (antigenic determinants, epitope), constituents of bacterial membranes, such as muramyldipeptide, lipopolysaccharide, an antibiotic, a hormone, a nucleoside, a nucleotide, a nucleic acid, an enzyme, an enzyme substrate, an enzyme inhibitor, biotin, avidin, polyethylene glycol, peptidic active compounds such as tuftsin; polylysine, an alkaloid, steroid, biogenic amine, vitamin or a toxin such as, for example, digoxin, phalloidin, amanitin or tetrodotoxin.

4. The process as claimed in one of the preceding claims, wherein the membrane anchor compound and the active compound are covalently bonded together via a crosslinker, for example via a polyethylene glycol.

5. The process as claimed in one or more of claims 1-4, wherein the peptide/membrane anchor linkage is produced by condensation, addition, substitution or oxidation, for example disulfide formation.

6. The use of the compounds as claimed in one or more of claims 1 to 4 for the preparation of conventional and monoclonal antibodies in vivo and in vitro.

7. The use of the compounds as claimed in one or more of claims 1 to 4 for the preparation of synthetic vaccines.

8. The use of the compounds as claimed in one or more of claims 1 to 4 for liposome preparations.

9. The use of the compounds as claimed in one or more of claims 1 to 4 for addition to human foodstuffs or animal feeds, and for addition to culture media for microorganisms and generally for cell cultures.

10. The use of a membrane anchor/active compound conjugate comprising at least one membrane anchor compound and at least one active compound covalently bonded to the membrane anchor compound(s), where the membrane anchor compound is a compound of the formula I: 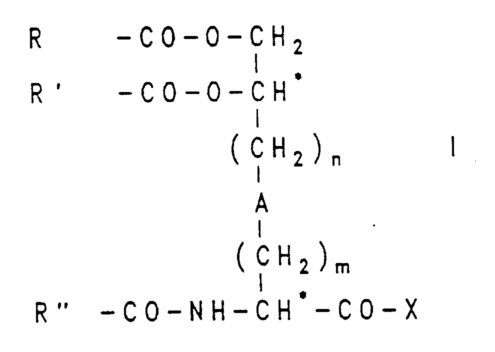 it being possible for A to be sulfur, oxygen, disulfide (-S-S-), methylene (-CH₂-) or -NH-; n being 0 to 5; m being 1 or 2; C* being an asymmetric carbon atom with R or S configuration; R, R' and R'' being identical or different and being an alkyl, alkenyl or alkynyl group having 7 to 25 carbon atoms or hydrogen, which can optionally be substituted by hydroxyl, amino, oxo, acyl, alkyl or cycloalkyl groups, for the preparation of a pharmaceutical for generating an immune response toward the active compound.

## Revendications (Revendications pour l'(les) Etat(s) contractant(s) suivant(s): BE, CH, DE, FR, GB, IT, LI, LU, NL, SE)

1. Conjugué substance active-composé d'ancrage à la membrane, caractérisé en ce qu'il consiste en au moins un composé d'ancrage à la membrane et au moins une substance active liée par covalence au(x) composé(s) d'ancrage à la membrane, le composé d'ancrage à la membrane étant un composé de formule générale I: 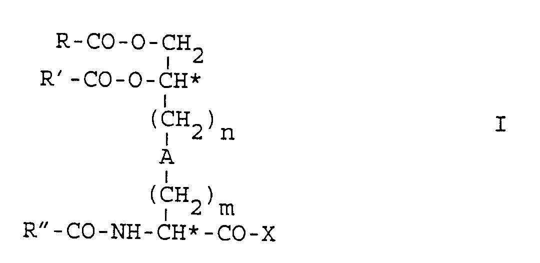 dans laquelle A peut être un atome d'oxygène ou de soufre ou un groupe disulfure (-S-S-), méthylène (-CH₂-) ou -NH-; n = 0 à 5; m = 1 ou 2; C* est un atome de carbone asymétrique à configuration R ou S; R, R', R'' sont identiques ou différents et représentent un atome d'hydrogène ou un groupe alkyle, alcényle ou alcynyle ayant de 7 à 25 atomes de carbone, qui peut éventuellement être substitué par des groupes hydoxy, amino, oxo, acyle, alkyle ou cycloalkyle, et X est une substance active ou un groupe substance active-espaceur, à l'exception des composés de formule I' 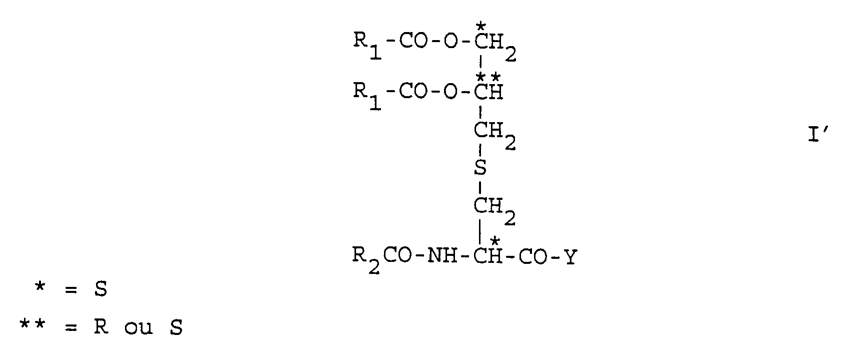 dans laquelle R₁ et R₂ représentent chacun un radical hydrocarboné ayant de 11 à 21 atomes de carbone, saturé ou insaturé, aliphatique ou mixte aliphatique-cycloaliphatique, éventuellement substitué par des fonctions oxygénées, et Y représente un aminoacide aliphatique naturel lié par une liaison peptidique, à groupe carboxy libre, estérifié ou amidé, ou une séquence d'aminoacides de 2 à 10 aminoacides aliphatiques naturels, dont le groupe carboxy terminal se trouve sous forme libre, estérifiée ou amidée, les centres d'asymétrie désignés par * ou ** ayant la configuration absolue S ou bien S ou R, et des mélanges des composés R et S épimères au niveau des atomes de C**.

2. Conjugué substance active-composé d'ancrage à la membrane selon la revendication 1, caractérisé en ce que le composé d'ancrage à la membrane est Pam₃Cys ou Pam₃Cys-Ser ou un Pam₃Cys-peptide comportant de 1 à 10 aminoacides.

3. Conjugué substance active-composé d'ancrage à la membrane selon la revendication 1 ou 2, caractérisé en ce que la substance active est un antigène, comme par exemple une séquence partielle à faible masse moléculaire d'une protéine ou d'un protéide, par exemple d'une glycoprotéine, d'une protéine d'enveloppe virale, d'une protéine de paroi cellulaire bactérienne ou d'une protéine de protozoaire (déterminant antigénique, épitope), des composants de membrane bactérienne, tels que le muramyldipeptide, un lipopolysaccharide, un antibiotique, une hormone, un nucléoside, un nucléotide, un acide nucléique, une enzyme, un substrat pour une enzyme, un inhibiteur d'enzyme, la biotine, l'avidine, le polyéthylèneglycol, des substances actives peptidiques telles que la tuftsine, la polylysine, un alcaloïde, un stéroïde, une amine biogène, une vitamine, une toxine comme par exemple la digoxine, la phalloïdine, l'amanitine ou la tétrodoxine.

4. Conjugué substance active-composé d'ancrage à la membrane selon l'une des revendications précédentes, caractérisé en ce que le composé d'ancrage à la membrane et la substance active sont liés l'un à l'autre par covalence, par l'intermédiaire d'un agent de liaison transversale, par exemple par l'intermédiaire d'un polyéthylèneglycol.

5. Procédé pour la préparation de composés d'ancrage à la membrane selon une ou plusieurs des revendication 1 à 4, caractérisé en ce que le peptide protégé par des groupes protecteurs, d'une façon connue en soi, sur les fonctions au niveau desquelles ne doit avoir lieu aucune réaction, est synthétisé à l'aide de procédés de couplages connus sur un support solide ou soluble, tel qu'un polymère (par exemple une résine selon Merrifield); en ce que les peptides synthétisés de cette façon, fixés sur le support, sont liés par covalence, par l'extrémité N-terminale ou des fonctions latérales du peptide, au composé d'ancrage à la membrane; en ce que le conjugué peptidique préparé de cette façon est isolé par élimination des groupes protecteurs et coupure, d'une façon connue en soi, de la liaison peptide/support, et on recueille ainsi le peptide d'ancrage à la membrane ou le conjugué substance active-composé d'ancrage à la membrane.

6. Procédé selon la revendication 5, caractérisé en ce que la liaison peptide/composé d'ancrage à la membrane est réalisée par condensation, addition, substitution, oxydation, par exemple formation d'une liaison disulfure.

7. Utilisation des composés selon une ou plusieurs des revendications 1 à 4, pour la production d'anticorps classiques et d'anticorps monoclonaux in vivo et in vitro.

8. Utilisation des composés selon une ou plusieurs des revendications 1 à 4, pour la fabrication de vaccins synthétiques.

9. Utilisation des composés selon une ou plusieurs des revendications 1 à 4, pour des compositions de liposomes.

10. Utilisation des composés selon une ou plusieurs des revendications 1 à 4, en tant qu'additif à des produits alimentaires ou des aliments pour animaux, dans le domaine humain et le domaine animal, ainsi que comme additif à des milieux de culture pour des micro-organismes et en général pour des cultures de cellules.

11. Utilisation d'un conjugué substance active-composé d'ancrage à la membrane, caractérisé en ce qu'il consiste en au moins un composé d'ancrage à la membrane et au moins une substance active liée par covalence au(x) composé(s) d'ancrage à la membrane, le composé d'ancrage à la membrane étant un composé de formule générale 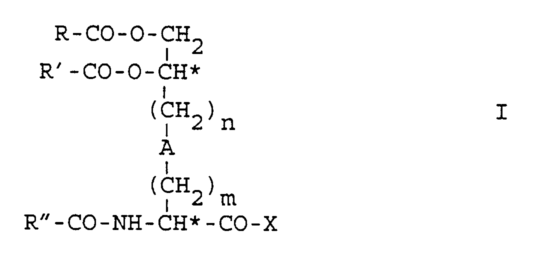 dans laquelle A peut être un atome d'oxygène ou de soufre ou un groupe disulfure (-S-S-), méthylène (-CH₂-) ou -NH-; n = 0 à 5; m = 1 ou 2; C* est un atome de carbone asymétrique à configuration R ou S; R, R', R'' sont identiques ou différents et représentent un atome d'hydrogène ou un groupe alkyle, alcényle ou alcynyle ayant de 7 à 25 atomes de carbone, qui peut éventuellement être substitué par des groupes hydoxy, amino, oxo, acyle, alkyle ou cycloalkyle, pour la fabrication d'un médicament destiné à engendrer une réponse immunitaire contre la substance active.

## Revendications (Revendications pour l'(les) Etat(s) contractant(s) suivant(s): AT)

1. Procédé pour la préparation d'un conjugué substance active-composé d'ancrage à la membrane, consistant en au moins un composé d'ancrage à la membrane et au moins une substance active liée par covalence au(x) composé(s) d'ancrage à la membrane, le composé d'ancrage à la membrane étant un composé de formule générale I: 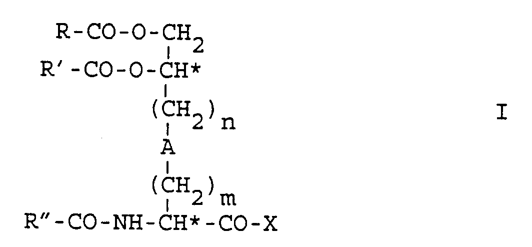 dans laquelle A peut être un atome d'oxygène ou de soufre ou un groupe disulfure (-S-S-), méthylène (-CH₂-) ou -NH-; n = 0 à 5; m = 1 ou 2; C* est un atome de carbone asymétrique à configuration R ou S; R, R', R'' sont identiques ou différents et représentent un atome d'hydrogène ou un groupe alkyle, alcényle ou alcynyle ayant de 7 à 25 atomes de carbone, qui peut éventuellement être substitué par des groupes hydoxy, amino, oxo, acyle, alkyle ou cycloalkyle, et X est une substance active ou un groupe substance active-espaceur, à l'exception des composés de formule I' 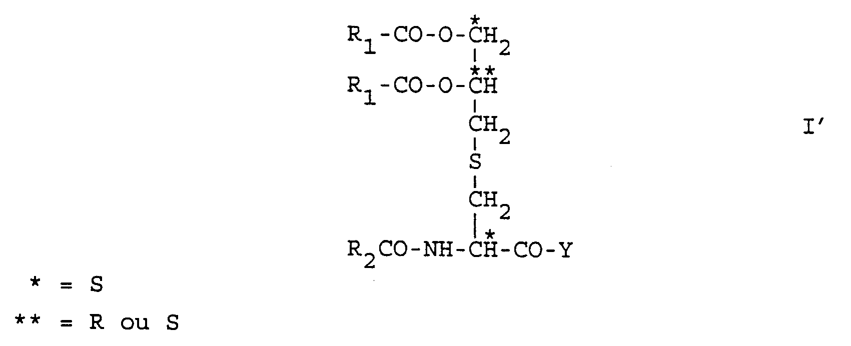 dans laquelle R₁ et R₂ représentent chacun un radical hydrocarboné ayant de 11 à 21 atomes de carbone, saturé ou insaturé, aliphatique ou mixte aliphatique-cycloaliphatique, éventuellement substitué par des fonctions oxygénées, et Y représente un aminoacide aliphatique naturel lié par une liaison peptidique, à groupe carboxy libre, estérifié ou amidé, ou une séquence d'aminoacides de 2 à 10 aminoacides aliphatiques naturels, dont le groupe carboxy terminal se trouve sous forme libre, estérifiée ou amidée, les centres d'asymétrie désignés par * ou ** ayant la configuration absolue S ou bien S ou R, et des mélanges des composés R et S épimères au niveau des atomes de C**, caractérisé en ce que le peptide protégé par des groupes protecteurs, d'une façon connue en soi, sur les fonctions au niveau desquelles ne doit avoir lieu aucune réaction, est synthétisé à l'aide de procédés de couplages connus sur un support solide ou soluble, tel qu'un polymère (par exemple une résine selon Merrifield) ; en ce que les peptides synthétisés de cette façon, fixés sur le support, sont liés par covalence, par l'extrémité N-terminale ou des fonctions latérales du peptide, au composé d'ancrage à la membrane; en ce que le conjugué peptidique préparé de cette façon est isolé par élimination des groupes protecteurs et coupure, d'une façon connue en soi, de la liaison peptide/support, et on recueille ainsi le peptide d'ancrage à la membrane ou le conjugué substance active-composé d'ancrage à la membrane.

2. Procédé selon la revendication 1, caractérisé en ce que le composé d'ancrage à la membrane est Pam₃Cys ou Pam₃Cys-Ser ou un Pam₃Cys-peptide comportant de 1 à 10 aminoacides.

3. Procédé selon la revendication 1 ou 2, caractérisé en ce que la substance active est un antigène, comme par exemple une séquence partielle à faible masse moléculaire d'une protéine ou d'un protéide, par exemple d'une glycoprotéine, d'une protéine d'enveloppe virale, d'une protéine de paroi cellulaire bactérienne ou d'une protéine de protozoaire (déterminant antigénique, épitope), des composants de membrane bactérienne, tels que le muramyldipeptide, un lipopolysaccharide, un antibiotique, une hormone, un nucléoside, un nucléotide, un acide nucléique, une enzyme, un substrat pour une enzyme, un inhibiteur d'enzyme, la biotine, l'avidine, le polyéthylèneglycol, des substances actives peptidiques telles que la tuftsine, la polylysine, un alkaloïde, un stéroïde, une amine biogène, une vitamine, une toxine comme par exemple la digoxine, la phalloïdine, l'amanitine ou la tétrodoxine.

4. Procédé selon l'une des revendications précédentes, caractérisé en ce que le composé d'ancrage à la membrane et la substance active sont liés l'un à l'autre par covalence, par l'intermédiaire d'un agent de liaison transversale, par exemple par l'intermédiaire d'un polyéthylèneglycol.

5. Procédé selon une ou plusieurs des revendications 1 à 4, caractérisé en ce que la liaison peptide/composé d'ancrage à la membrane est réalisée par condensation, addition, substitution, oxydation, par exemple formation d'une liaison disulfure.

6. Utilisation des composés selon une ou plusieurs des revendications 1 à 4, pour la production d'anticorps classiques et d'anticorps monoclonaux in vivo et in vitro.

7. Utilisation des composés selon une ou plusieurs des revendications 1 à 4, pour la fabrication de vaccins synthétiques.

8. Utilisation des composés selon une ou plusieurs des revendications 1 à 4, pour des compositions de liposomes.

9. Utilisation des composés selon une ou plusieurs des revendications 1 à 4, en tant qu'additif à des produits alimentaires ou des aliments pour animaux, dans le domaine humain et le domaine animal, ainsi que comme additif à des milieux de culture pour des micro-organismes et en général pour des cultures de cellules.

10. Utilisation d'un conjugué substance active-composé d'ancrage à la membrane, caractérisé en ce qu'il consiste en au moins un composé d'ancrage à la membrane et au moins une substance active liée par covalence au(x) composé(s) d'ancrage à la membrane, le composé d'ancrage à la membrane étant un composé de formule générale 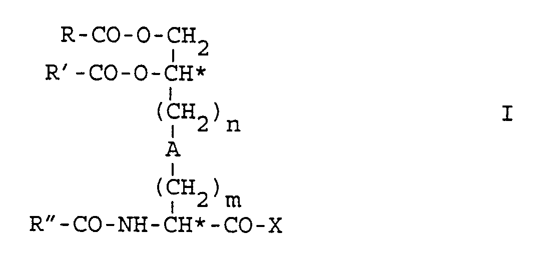 dans laquelle A peut être un atome d'oxygène ou de soufre ou un groupe disulfure (-S-S-), méthylène (-CH₂-) ou -NH-; n = 0 à 5; m = 1 ou 2; C* est un atome de carbone asymétrique à configuration R ou S; R, R', R'' sont identiques ou différents et représentent un atome d'hydrogène ou un groupe alkyle, alcényle ou alcynyle ayant de 7 à 25 atomes de carbone, qui peut éventuellement être substitué par des groupes hydoxy, amino, oxo, acyle, alkyle ou cycloalkyle, pour la fabrication d'un médicament destiné à engendrer une réponse immunitaire contre la substance active.
